# EUROPEAN PATENT APPLICATION

(11) **EP 4 726 045 A1**
(43) Date of publication of application: **15.04.2026**
(21) Application number: 24818609.0
(22) Date of filing: 03.06.2024
(51) Int. Cl.: C12N 15/12, C12N 15/864, C12N 7/01, A61K 48/00, A61K 38/17, A61P 27/02

(54) **EXPRESSION CASSETTE COMBINATION AND USE THEREOF**

(30) Priority: 07.06.2023 CN 202310670971
(71) Applicant: Peking University Third Hospital (The Third Clinical Medical School of Peking University), Beijing 100191 (CN); Chigenovo Co., Ltd., Beijing 102206 (CN)
(72) Inventor: YANG, Liping, Beijing 100191 (CN); CHEN, Shaohong, Beijing 102206 (CN); MENG, Xin, Beijing 102206 (CN); QIAO, Jing, Beijing 102206 (CN); YANG, Jierong, Beijing 102206 (CN); ZHONG, Xiancheng, Beijing 102206 (CN)
(74) Representative: Hoffmann Eitle
(86) International application number: PCT/CN2024/097025
(87) International publication number: WO 2024/251077

(57) **Abstract**

An expression cassette combination and use thereof. The expression cassette combination comprises the first expression cassette and the second expression cassette, wherein the first expression cassette can express a fusion protein of the N-terminal truncated form of ABCA4 protein and the N-terminal of intein, and the second expression cassette can express a fusion protein of the C-terminal truncated form of ABCA4 protein and the C-terminal of intein. When expressed, the N-terminal truncated form of the ABCA4 protein expressed by the first expression cassette and the C-terminal truncated form of the ABCA4 protein expressed by the second expression cassette can form a complete full-length ABCA4 protein through the cleavage function of the N-terminal and C-terminal of the intein.

## Description

### TECHNICAL FIELD

The present application relates to biopharmaceutical field, specifically to an expression cassette combination and use thereof.

### BACKGROUND OF THE INVENTION

ABCA4 is a lipid inward flippase in retinal photoreceptor cells or retinal pigment epithelial cells, which can transport a lipid derivative formed by phosphatidylethanolamine (PE) and all-trans retinoic aldehyde (ATR), i.e. N-retinylidene-phosphatidylethanolamine (NRPE), from the lumen side of the outer segment membranous disk to the cytoplasmic side. Subsequently, NRPE is hydrolyzed into ATR and PE, and the ATR therein is catalyzed by retinaldehyde reductase in the cytoplasm to form all-trans retinol (ATRol), thus returning to the visual cycle. If the lipid transportation function of ABCA4 is impaired, it will cause excessive accumulation of ATR and NRPE on the lumen side of the outer segment membranous disk. The excessive accumulation of ATR and NRPE will further irreversibly form toxic bisretinoid derivatives, ultimately causing a series of retinal degenerative diseases, including the most common hereditary macular degeneration diseases, age-related macular degeneration, retinitis pigmentosa, and cone-rod dystrophy, etc. The full length of human ABCA4 CDS is 6.7 kb, encoding 2273 amino acids.

Inteins refer to some peptide fragments present inside the peptide chain of certain protein precursors. When transformed into mature proteins, they are excised through non-enzymatic transpeptidation reactions, and the corresponding exteins are retained in the mature protein. Inteins have been widely used in protein purification, protein labeling, and the splicing expression of larger protein fragments.

CN115074369A utilizes an intein dual AAV vector strategy to express ABCA4 protein. It discloses that the most critical aspects of the split intein dual AAV vector strategy are the intein sequence and the cleavage site, because these will greatly affect the cleavage efficiency of the target protein, as well as the tertiary structure and post-translational modification of the target protein, and further affect protein function. In addition, since the split intein dual AAV vectors express the N-terminal and C-terminal of the ABCA4 protein respectively, those N-terminal and C-terminal by-products uneffectively spliced have a potential safety risk. In CN115074369A, a full-length ABCA4 protein is cleaved into two fragments with p1 150Cys as the cleavage site, and the intein used is the Rma intein or the Npu intein.

CN115698307A discloses inteins and use thereof, in which engineered split inteins and their combinations with degradation signals (destabilizing domains, degrons) are used to reconstruct large genes. One of the main limitations of the above use of inteins disclosed in CN115698307A is the accumulation of protein intermediates (N-extein-IntN and IntC-C-extein) and the excised split inteins. Those skilled in the art could understand that the accumulation of these unwanted proteins may cause adverse side effects (immune responses).

### BRIEF SUMMARY OF THE INVENTION

To address above problems, the present inventors conducted an in-depth research and found that by selecting a specific cleavage site of the ABCA4 protein, obtaining the N-terminal truncated form and C-terminal truncated form of the ABCA4 protein, and then connecting them to the N-terminal and C-terminal portions of a specific intein, respectively, the first expression cassette expressing a fusion protein of the N-terminal truncated form of the ABCA4 protein and the N-terminal of intein and the second expression cassette expressing a fusion protein of the C-terminal truncated form of the ABCA4 protein and the C-terminal of the intein are obtained. The first expression cassette and the second expression cassette are expressed in cells with high efficiency to form a complete ABCA4 protein; AAV5 and AAV8 viruses can be successfully packaged, and can be effectively expressed in mouse retinal cells, and restore the retinal function of ABCA4 KO mice; the above viruses co-infect IPSC-RPE or IPSC-PRC cells derived from patients with Stargardt's disease, can restore the retinol conversion function of the cells, and reduce the deposition of A2E (retinoid dimer (N-retinyl-N-retinylamine)); and when using the GP41-1 intein, the amount of the intein protein produced by the above expression cassette is less, having a potential advantage of low immune response.

Particularly, the present inventors unexpectedly found that by selecting the GP41-1 intein, not only can the highest expression efficiency of the complete ABCA4 protein be obtained as compared to other inteins, but also less additional inteins are produced, thus having less unwanted immunogenicity and side effects when used for *in vivo* treatment.

In addition, the present inventors also unexpectedly found that in the case of using the same type of intein (such as GP41-1), an excellent expression efficiency of the complete ABCA4 protein can be obtained by selecting a specific cleavage site of the ABCA4 protein (i.e., the 1224Cys site starting from the N-terminus).

Therefore, in one aspect, the present invention provides an expression cassette combination, which comprises the first expression cassette and the second expression cassette, wherein the first expression cassette is capable of expressing a fusion protein of an N-terminal truncated form of ABCA4 protein and the N-terminal of an intein, and the second expression cassette is capable of expressing a fusion protein of a C-terminal truncated form of ABCA4 protein and the C-terminal of the intein, wherein when expressed, the N-terminal truncated form of ABCA4 protein expressed by the first expression cassette and the C-terminal truncated form of ABCA4 protein expressed by the second expression cassette can form a complete full-length ABCA4 protein through the cleavage function of the N-terminal and C-terminal of the intein.

In certain embodiments, the intein is selected from the group consisting of: GP41-1 (SEQ ID NO: 5), DnaE (SEQ ID NO: 4), GP41-8 (SEQ ID NO: 6), NrdJ-1 (SEQ ID NO: 7), IMPDH-1 (SEQ ID NO: 8), SspGyrB (SEQ ID NO: 9) and Rma (SEQ ID NO: 60), preferably GP41-1.

In certain embodiments, the complete ABCA4 protein is a human ABCA4 protein (SEQ ID NO: 1), and the N-terminal truncated form of ABCA4 protein expressed by the first expression cassette and the C-terminal truncated form of ABCA4 protein expressed by the second expression cassette are the two corresponding N-terminal and C-terminal truncated forms obtained by truncating the complete ABCA4 protein at the following amino acid (Cys) sites starting from the N-terminus of the ABCA4 protein: 1224, 1140, 1150, or 1188, preferably 1188 or 1224.

In certain embodiments, the N-terminal truncated form and the C-terminal truncated form are selected from the following combinations:
the amino acids at positions 1-1223 and 1224-2273 from the N-terminus of the ABCA4 protein (SEQ ID NO: 1);
the amino acids at positions 1-1139 and 1140-2273 from the N-terminus of the ABCA4 protein (SEQ ID NO: 1);
the amino acids at positions 1-1149 and 1150-2273 from the N-terminus of the human ABCA4 protein (SEQ ID NO: 1); or
the amino acids at positions 1-1187 and 1188-2273 from the N-terminus of the ABCA4 protein (SEQ ID NO: 1).

In certain embodiments, the N-terminal of intein and the C-terminal of the intein are selected from the group consisting of:
1) The N-terminal of GP41-1 (SEQ ID NO: 17) and the C-terminal of GP41-1 (SEQ ID NO: 18);
2) The N-terminal of DnaE (SEQ ID NO: 15) and the C-terminal of DnaE (SEQ ID NO: 16);
3) The N-terminal of GP41-8 (SEQ ID NO: 19) and the C-terminal of GP41-8 (SEQ ID NO: 20);
4) The N-terminal of NrdJ-1 (SEQ ID NO: 21) and the C-terminal of NrdJ-1 (SEQ ID NO: 22);
5) The N-terminal of IMPDH-1 (SEQ ID NO: 23) and the C-terminal of IMPDH-1 (SEQ ID NO: 24);
6) The N-terminal of SspGyrB (SEQ ID NO: 25) and the C-terminal of SspGyrB (SEQ ID NO: 26)
7) The N-terminal of Mja-KlbA (SEQ ID NO: 27) and the C-terminal of Mja-KlbA (SEQ ID NO: 28);
8) The N-terminal of NpuSsp (SEQ ID NO: 29) and the C-terminal of NpuSsp (SEQ ID NO: 30); and
9) The N-terminal of Rma (SEQ ID NO: 61) and the C-terminal of Rma (SEQ ID NO: 62).

In certain embodiments, the intein is GP41-1 and the N-terminal truncated form and C-terminal truncated form of the ABCA4 protein are the two corresponding N-terminal truncated form and C-terminal truncated form obtained by truncating at the 1224th amino acid (Cys) site starting from the N-terminus of the ABCA4 protein, i.e., the 1st to 1223rd amino acids and the 1224th to 2273rd amino acids of the human ABCA4 protein (SEQ ID NO: 1) starting from the N-terminus.

In certain embodiments, the intein is GP41-1 and the N-terminal truncated form and C-terminal truncated form of the ABCA4 protein are the corresponding N-terminal truncated form and C-terminal truncated form obtained by truncating at the 1188th amino acid (Cys) site starting from the N-terminus of the ABCA4 protein, i.e., the 1st to 1187th amino acids and the 1188th to 2273rd amino acids of the human ABCA4 protein (SEQ ID NO: 1) starting from the N-terminus.

In certain embodiments, the codon for expressing the full-length ABCA4 protein is an optimized codon, and has a sequence as shown in SEQ ID NO: 3. In certain embodiments, the codons for expressing the N-terminal truncated form and the C-terminal truncated form of the ABCA4 protein are optimized codons, and the combined (merged/linked) sequence of the two is the coding sequence of the full-length ABCA4 protein as shown in SEQ ID NO: 3. It is readily understood by those skilled in the art that codon optimization can be sequence optimization for the full-length ABCA4, or for the codon optimization of the N-terminal truncated form and C-terminal truncated form sequences, respectively.

In certain embodiments, the first tag is connected to the C-terminus of the first expression cassette, and/or the second tag is connected to the C-terminus of the second expression cassette, wherein the first tag and the second tag may be different. The first tag and the second tag are independently selected from the group consisting of: hemagglutinin (HA) tag, Flag tag, V5 tag, Myc tag, His tag, 1D4 tag (Rho1D4), and combinations thereof, preferably the first tag is a hemagglutinin (HA) tag (SEQ ID NO: 12) and/or the second tag is a Flag tag (SEQ ID NO: 13).

In some embodiments, the N-terminus or C-terminus of the intein is additionally connected to a degradation signal either directly or indirectly through a linker. Preferably, the degradation signal is selected from polypeptides related to DHFR (dihydrofolate reductase), FKBP (FK506-binding protein), FRB (FKBP-rapamycin binding protein), and PDE5 (phosphodiesterase type 5), as well as degradation signals of the endoplasmic reticulum-associated degradation (ERAD) pathway. More preferably, the degradation signal is ecDHFR (SEQ ID NO: 14).

In certain embodiments, the first expression cassette and/or the second expression cassette comprises a promoter and a terminator for expressing the fusion protein, and optionally comprises an enhancer and an intron for enhancing the expression of the fusion protein and operably linked to the fusion protein.

In certain embodiments, the promoter is a constitutive promoter or an inducible promoter, preferably selected from the group consisting of: CAG promoter (hybrid CMV early enhancer/chicken β-actin promoter, also known as CAGGS promoter, CB promoter or CBA promoter), human β-actin promoter, small CBA (smCBA) promoter, CBS promoter or CBh promoter, elongation factor 1α short (EFS) promoter, elongation factor 1α (EF-1α) promoter, CMV promoter, PGK promoter, UBC promoter, GUSB promoter, UCOE promoter, VMD2 (also known as BEST1) promoter, ABCA4 self-promoter, RPE65 promoter, OPEFS promoter, hGRK1 promoter or smCAG promoter, more preferably OPEFS promoter (SEQ ID NO: 47), hGRK1 promoter (SEQ ID NO: 48), smCAG promoter (SEQ ID NO: 49), enOPEFS promoter (SEQ ID NO: 67), enhGRK1 (enRK) promoter (SEQ ID NO: 68), hGRK1-b-globin-intron promoter (SEQ ID NO: 69) or enhGRK1-b-globin-intron promoter (SEQ ID NO: 70).

In certain embodiments, the terminator is selected from the group consisting of: SV40 terminator (SEQ ID NO: 50), BGH terminator (SEQ ID NO: 51), WPRE terminator (such as WPRE-3 terminator (SEQ ID NO: 52)), WPRE-SV40 terminator (SEQ ID NO: 56), WPRE-BGH terminator, B-globin-polyA terminator (SEQ ID NO: 65), or sNRP1 terminator (SEQ ID NO: 66).

In certain embodiments, the enhancer is the CMV enhancer (SEQ ID NO: 53). In certain embodiments, the intron is the intron from rhesus monkey beta herpesvirus-3 (MHV-3) (SEQ ID NO:54) or the SV40 intron (SEQ ID NO:55).

In certain embodiments, the first expression cassette comprises, sequentially from the 5'-end: an optional enhancer, a promoter, an optional intron, a nucleotide coding sequence of the N-terminal truncated form of the ABCA4 protein, a nucleotide coding sequence of the N-terminal of intein, an optional degradation signal, a nucleotide coding sequence of an optional tag, and a terminator, and/or the second expression cassette comprises, sequentially from the 5'-end: an optional enhancer, a promoter, an optional intron, a nucleotide coding sequence of the C-terminal of the intein, an optional degradation signal, a nucleotide coding sequence of the C-terminal truncated form of the ABCA4 protein, a nucleotide coding sequence of an optional tag, and a terminator. The nucleotide coding sequence of the N-terminal truncated form of the ABCA4 protein, the nucleotide coding sequence of the N-terminal of intein, the nucleotide coding sequence of the C-terminal of the intein, the nucleotide coding sequence of the C-terminal truncated form of the ABCA4 protein, the nucleotide coding sequence of an optional tag, and an optional degradation signal sequence are operably linked to the promoter/enhancer.

In certain embodiments, the first expression cassette and/or the second expression cassette exist in a form selected from the group consisting of: isolated nucleic acid molecules, liposomes, and/or exosomes.

In certain embodiments, the first expression cassette and/or the second expression cassette exist in the form of a plasmid.

In certain embodiments, the first expression cassette and/or the second expression cassette are viral vectors.

In certain embodiments, the viral vector is an adeno-associated virus (AAV) of the same or different serotypes, and the AAV is selected from the group consisting of: AAV1, AAV2, AAV3, AAV4, AAV5, AAV6, AAV7, AAV8, AAV9, AAV10, AAV11, AAV12,, AAV2/5, AAV2/8, AAV2/1, AAV2/9, AAV2/6, AAV2/4, AAV2/6, AAV5/2, AAV8/1, AAV8/2, AAV2/7, AAV2/12, AAV2/10, AAV-DJ, AAV-DJ/8, AAV-DJ/9, AAV8-Y447,733F or AAVtYF, preferably AAV5, AAV8, AAV8-Y447,733F, AAVtYF or AAV2.7m8.

In certain embodiments, the expression cassette combination comprises the following combinations of elements:
1) The promoter is hGRK1, the truncation site of the N-terminal truncated form and C-terminal truncated form is 1188, the intein is Rma, and the terminator is SV40;
2) The promoter is enRK, the truncation site of the N-terminal truncated form and C-terminal truncated form is 1188, the intein is GP41-1, and the terminator is SV40;
3) The promoter is enRK, the truncation site of the N-terminal truncated form and C-terminal truncated form is 1188, the intein is Rma, and the terminator is B-globin-polyA;
4) The promoter is enRK, the truncation site of the N-terminal truncated form and C-terminal truncated form is 1188, the intein is GP41-1, and the terminator is B-globin-polyA;
5) The promoter is hGRK1, the truncation site of the N-terminal truncated form and C-terminal truncated form is 1188, the intein is GP41-1, and the terminator is SV40;
6) The promoter is enRK, the truncation site of the N-terminal truncated form and C-terminal truncated form is 1150, the intein is GP41-1, and the terminator is SV40; or
7) The promoter is enRK-b-globin intron, the truncation site of the N-terminal truncated form and C-terminal truncated form is 1188, the intein is GP41-1, and the terminator is B-globin-polyA.

In another aspect, the present invention provides a kit comprising the above-described expression cassette combination.

In another aspect, the present invention provides the use of the expression cassette combination or kit of the present invention in the preparation of a medicament for treating a disease, the disease comprising diseases caused by ABCA4 mutations.

In certain embodiments, the disease comprises hereditary retinal diseases.

In certain embodiments, the disease comprises hereditary macular degeneration diseases, age-related macular degeneration, retinitis pigmentosa and/or cone-rod dystrophy.

Those skilled in the art can easily gain insights into other aspects and advantages of this application from the following detailed description. Only exemplary embodiments of this application are shown and described in the following detailed description. As those skilled in the art will recognize, the disclosure of this application enables one of skill in the art to modify the disclosed specific embodiments without departing from the spirit and scope of the invention. Accordingly, the descriptions in the drawings and the specification of the application are merely exemplary and not restrictive.

### BRIEF DESCRIPTION OF THE DRAWINGS

From the following detailed description in conjunction with the drawings, the above features and advantages of the present invention will become more apparent, wherein:
FIG. 1 shows the protein expression level of codon-optimized ABCA4;
FIG. 2 shows a schematic diagram of forming a complete ABCA4 protein according to the intein dual-expression cassette strategy of the present invention;
FIG. 3 shows the ABCA4 protein expression efficiency of different types of split inteins in the ABCA4 protein expression strategy of the present invention;
FIG. 4 shows the splicing efficiency of inteins (GP41-1 and Rma) to form full-length ABCA4;
FIG. 5 shows that different truncation sites of the ABCA4 protein result in different ABCA4 protein expression efficiencies in the ABCA4 protein expression strategy of the present invention;
FIG. 6 shows that the dual-expression cassette comprising a degradation peptide can form a complete ABCA4 protein, and the degradation signal causes a significant degradation of the intein;
FIG. 7 shows that the expression cassette constructed in Example 5 can form a complete ABCA4 protein in 293T cells;
FIG. 8 shows that the expression cassette constructed in Example 6 can restore the ERG amplitude level in ABCA4 KO mice;
FIG. 9 shows the A2E content in the eyeball retina tissue of mice after injecting the expression cassette constructed in Example 6;
FIG. 10 shows that the expression cassette constructed in Example 6 can successfully express a complete ABCA4 protein in ABCA4 KO mice;
FIG. 11 shows that each of various cleavage sites can effectively express the full-length ABCA4 protein *in vivo;*
FIG. 12 shows that each of various stop codons can effectively express the full-length ABCA4 protein *in vivo;*
FIG. 13 shows that each of various promoters can effectively express the full-length ABCA4 protein in the mouse retina;
Figure 14 shows that each of expression cassettes having various combinations of elements can effectively express the full-length ABCA4 protein in the retinas of model mice;
Figure 15 shows the expression of full-length ABCA4 protein in the retinas of mice with different serotypes of AAVs;
Figure 16 shows that the content of the additional small peptides produced by the split intein GP41-1 is less; and
Figure 17 shows the ERG, fundus OCT, and FP test results after subretinal injection of AAV8-GP41-1 and AAV8-DnaE into ABCA4 KO mice, respectively.

### DETAILED DESCRIPTION OF THE INVENTION

Unless otherwise indicated, the terms used herein have the general technical meanings understood by those skilled in the art. For definitions and terms in this field, one skilled artisan is particularly recommended to refer to Sambrook et al., Molecular Cloning: A Laboratory Manual, 2nd edition, Cold Spring Harbor Press, Plainsview, New York (1989); and Ausubel et al., Current Protocols in Molecular Biology (Supplement 47), John Wiley & Sons, New York (1999).

The term "first expression cassette" generally comprises an exogenous DNA sequence operably linked to a promoter or other regulatory sequences sufficient to direct the transcription of a gene of interest. In the present application, the expression cassette generally refers to such a nucleic acid construct: when introduced into a host cell, it results in the transcription and/or translation of RNA or polypeptide, respectively. The expression cassette can have a series of specialized nucleic acid elements that allow the transcription of a specific nucleic acid in a target cell. The expression cassette can be incorporated into a plasmid, chromosome, mitochondrial DNA, plasmid DNA, virus, or nucleic acid fragment. The expression cassette may comprise untranslated or non-translatable antisense or sense constructs. In the case of expressing a transgenic gene and suppressing an endogenous gene (e.g., by antisense, RNAi, or sense suppression), those skilled in the art will recognize that the inserted polynucleotide sequence does not need to be the same, but can merely be substantially the same as the sequence of its source gene. The expression cassette may comprise a polynucleotide construct of a transcription factor operably linked to a promoter, and the promoter can be the promoter of a gene regulated by the transcription factor. In the present application, the first expression cassette can express a fusion protein (e.g., a fusion protein of the N-terminal truncated form of ABCA4 protein and the N-terminal of an intein).

The term "second expression cassette" generally refers to an expression cassette that is not completely the same as the first expression cassette. For example, the second expression cassette can express a protein different from the protein expressed by the first expression cassette (e.g., a fusion protein of the C-terminal truncated form of ABCA4 protein and the C-terminal of the intein).

The term "expression cassette combination" generally refers to a combination comprising at least one expression cassette. For example, the expression cassette combination described in the present application may comprise the first expression cassette and the second expression cassette.

In the present application, the term "operably linked" generally refers to placing regulatory sequences necessary for the expression of a coding sequence in an appropriate position relative to the coding sequence so as to carry out the expression of the coding sequence. For example, the first nucleic acid sequence is operably linked to the second nucleic acid sequence when the first nucleic acid sequence is in a functional relationship with the second nucleic acid sequence. In certain embodiments, it may represent the arrangement of a coding sequence and a transcriptional control element(s) in an expression vector. The control elements may comprise a promoter, an enhancer, and a termination element. For example, if a promoter affects the transcription or expression of a coding sequence, the promoter is operably linked to the coding sequence. In certain embodiments, "operably linked" may also refer to a target gene being linked into a vector such that the transcriptional and translational control sequences within the vector perform their intended functions of regulating the transcription and translation of the target gene.

The term "ABCA4 gene" generally refers to the gene encoding rim protein or RmP. The ABCA4 gene is also referred to as the ABCR gene. This gene was first cloned and identified as a gene that can cause Stargardt disease, an autosomal recessive genetic disease that can lead to macular degeneration. The NCBI Entrez Gene accession number of the human ABCA4 gene is 24. In one embodiment, the nucleotide sequence of the ABCA4 gene is as shown in SEQ ID NO:2.

The term "complete (full-length) ABCA4 protein" generally refers to the member 4 of the ATP-binding cassette subfamily A with a complete structure. The full-length ABCA4 protein can be encoded by the human ABCA4 gene. ABCA4 is a lipid inward flippase in retinal photoreceptor cells or retinal pigment epithelial cells, which can transport a lipid derivative formed by phosphatidylethanolamine (PE) and all-trans retinal (ATR), i.e., N-retinylidene-phosphatidylethanolamine (NRPE), from the lumen side of the outer segment membranous disc to the cytoplasmic side. Subsequently, NRPE is hydrolyzed into ATR and PE, and the ATR therein is catalyzed by retinaldehyde reductase in the cytoplasm to form all-trans retinol (ATRol), thus returning to the visual cycle. The UniProt accession number of the human full-length ABCA4 protein is P78363. In one embodiment, the sequence of the human full-length ABCA4 protein is as shown in SEQ ID NO:1.

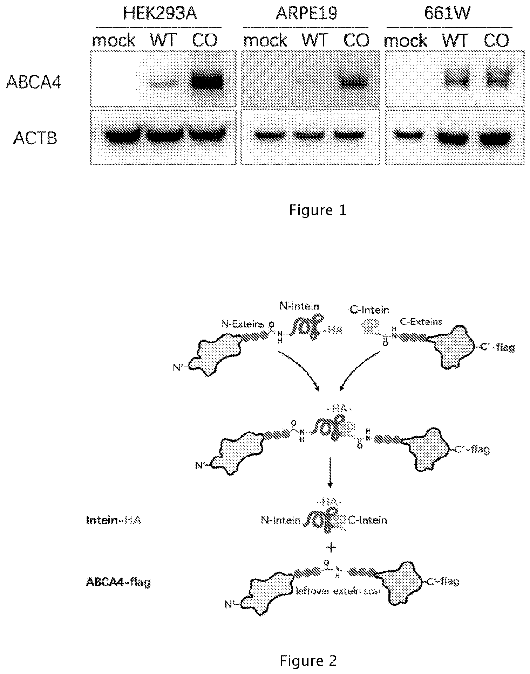

The term "N-terminal truncated form of ABCA4 protein" generally refers to the N-terminal truncated form of the full-length human ABCA4 protein. For example, the N-terminal truncated form of the ABCA4 protein may comprise the amino acid sequence shown by amino acids 1-1139, 1-1149, 1-1187, 1-1223, 1-1325, 1-1160, 1-1220, 1-1280, or 1-1347 starting from the N-terminus of the full-length human ABCA4 protein. For example, the amino acid sequence of the N-terminal truncated form of the ABCA4 protein may be as shown by amino acids 1-1325 starting from the N-terminus of the full-length human ABCA4 protein. In the present application, the N-terminal truncated form of the ABCA4 protein and/or the C-terminal truncated form of the ABCA4 protein may correspond to the blue and green structures in Figure 1 of Nature Communications volume 12, Article number: 3853 (2021), respectively.

In the present application, the N-terminal truncated form of the ABCA4 protein may comprise the following domains at the N-terminal of the ABCA4 protein: transmembrane domains TMD1-TMD6, extracellular domains ECD1, IH1, IH2, EH1, EH2, NBD1, and/or R1.

In the present application, the N-terminal truncated form of the ABCA4 protein may successively comprise, starting from the N-terminus: IH1, TMD1, ECD1, TMD2, IH2, TMD3, TMD4, TMD5, EH1, EH2, TMD6, NBD1, and/or R1. For example, the N-terminal truncated form of the ABCA4 protein may successively comprise, starting from the N-terminus: IH1, TMD1, ECD1, TMD2, IH, TMD3, TMD4, TMD5, EH1, EH2, TMD6, and/or NBD1.

In the present application, the first expression cassette may comprise a nucleotide sequence encoding the N-terminal truncated form of the ABCA4 protein.

The term "C-terminal truncated form of ABCA4 protein" generally refers to the C-terminal truncated form of the full-length human ABCA4 protein. For example, the C-terminal truncated form of the ABCA4 protein may comprise the amino acid sequence shown by amino acids 1140-2273, 1150-2273, 1188-2273, 1224-2273, 1326-2273, 1348-2273, 1369-2273, or 1348-2170 starting from the N-terminus of the full-length human ABCA4 protein. For example, the amino acid sequence of the C-terminal truncated form of the ABCA4 protein may be as shown by amino acids 1326-2273 starting from the N-terminus of the full-length human ABCA4 protein.

In present application, the first expression cassette may comprise a promoter. In present application, the promoter may comprise a constitutive promoter and/or an inducible promoter. For example, the promoter may be selected from the group consisting of: CAG promoter (hybrid CMV early enhancer/chicken β-actin promoter, also known as CAGGS promoter, CB promoter or CBA promoter), human β-actin promoter, small CBA (smCBA) promoter, CBS promoter or CBh promoter, elongation factor 1α short (EFS) promoter, elongation factor 1α (EF-1α) promoter, CMV promoter, PGK promoter, UBC promoter, GUSB promoter, UCOE promoter, VMD2 (also known as BEST1) promoter, ABCA4 self-promoter, RPE65 promoter, OPEFS promoter, hGRK1 promoter or smCAG promoter, more preferably OPEFS promoter (SEQ ID NO: 47), hGRK1 promoter (SEQ ID NO: 48) or smCAG promoter (SEQ ID NO: 49).

In present application, the C-terminal truncated form of the ABCA4 protein may comprise the domains at the C-terminal of the ABCA4 protein: transmembrane regions TMD7 to TMD12, extracellular domains ECD1, IH3, IH4, EH3, EH4, NBD2 and/or R2.

In present application, the C-terminal truncated form of the ABCA4 protein may sequentially comprise, from the N-terminus: IH3, TMD7, ECD2, TMD8, IH4, TMD9, TMD10, TMD11, EH3, EH4, TMD12, NBD2 and/or R2. For example, the C-terminal truncated form of the ABCA4 protein may sequentially comprise, from the N-terminus: IH3, TMD7, ECD2, TMD8, IH4, TMD9, TMD10, TMD11, EH3, EH4, TMD12 and/or NBD2.

In present application, the second expression cassette may comprise a nucleotide sequence encoding the C-terminal truncated form of the ABCA4 protein.

In present application, the second expression cassette may comprise a promoter. In present application, the promoter may comprise a constitutive promoter and/or an inducible promoter.

For example, the promoter is selected from the group consisting of: CAG promoter (hybrid CMV early enhancer/chicken β-actin promoter, also known as CAGGS promoter, CB promoter or CBA promoter), human β-actin promoter, small CBA (smCBA) promoter, CBS promoter or CBh promoter, elongation factor 1α short (EFS) promoter, elongation factor 1α (EF-1α) promoter, CMV promoter, PGK promoter, UBC promoter, GUSB promoter, UCOE promoter, VMD2 (also known as BEST1) promoter, ABCA4 self-promoter, RPE65 promoter, OPEFS promoter, hGRK1 promoter or smCAG promoter, more preferably OPEFS promoter (SEQ ID NO: 47), hGRK1 promoter (SEQ ID NO: 48) or smCAG promoter (SEQ ID NO: 49).

Herein, the N-terminal truncated form of the ABCA4 protein and the C-terminal truncated form of the ABCA4 protein may correspond to the two N-terminal and C-terminal truncated forms obtained by truncating the complete ABCA4 protein at a specific site (such as Cys). In other words, the N-terminal truncated form of the ABCA4 protein and the C-terminal truncated form of the ABCA4 protein in the present invention can be spliced from the N-terminal to the C-terminal direction (for example, through the shearing functions of the N-terminal and C-terminal of the inteins connected to each other) to form a complete ABCA4 protein, so as to exert its biological function. The preferred truncation positions are the following amino acid (Cys) sites starting from the N-terminus of the ABCA4 protein: 1224, 1140, 1150 or 1188, more preferably 1188 or 1224. For example, when the truncation position is at the 1188 site, the N-terminal truncated form of the ABCA4 protein is the amino acids at positions 1-1187 starting from the N-terminus, and the corresponding C-terminal truncated form is the amino acids at positions 1188-2273 starting from the N-terminus. For example, when the truncation position is at the 1224 site, the N-terminal truncated form of the ABCA4 protein is the amino acids at positions 1-1223 starting from the N-terminus, and the corresponding C-terminal truncated form is the amino acids at positions 1224-2273 starting from the N-terminus.

In present application, the molar ratio of the N-terminal truncated form of the ABCA4 protein expressed by the first expression cassette to the C-terminal truncated form of the ABCA4 protein expressed by the second expression cassette is about 3:1 to 1:3. For example, the molar ratio of the N-terminal truncated form of the ABCA4 protein to the C-terminal truncated form of the ABCA4 protein expressed by the second expression cassette may be about 3:1 to 1:3, about 3:1 to 1:2, about 3:1 to 1:1, about 3:1 to 2:1, about 2:1 to 1:3, about 2:1 to 1:2, about 2:1 to 1:1, about 1:1 to 1:3, about 1:2 to 1:1, about 1:1.

The term "biological function" generally refers to the activity that is native to the biological entity being tested or the intended activity of said biological entity, such as the native activity of a cell, protein, or the like. Ideally, *in vitro* functional assays may be used to test for the presence of a biological function.

In the present application, the biological functions of the full-length ABCA4 protein may include cell localization function; ATP hydrolase activity; expression in photoreceptor cells, and/or, reduction of retinal A2E deposition.

As used herein, the term "intein" refers to a naturally occurring or artificially constructed polypeptide sequence that is capable of catalyzing a protein splicing reaction in which an intein sequence is excised from a precursor protein and the flanking sequences (N-extein and C-extein) are joined via peptide bonds. A list of known inteins is published at http://www.inteins.com. Table 1 of CN114698379A summarizes hundreds of naturally occurring inteins. In one embodiment, the intein of the present invention is a split intein. In the present invention, the intein may be selected from, but is not limited to, the group consisting of: GP41-1, DnaE, GP41-8, NrdJ-1, IMPDH-1, SspGyrB, Mja-KlbA, NpuSsp, and Rma. In one embodiment, the sequences of these inteins are shown in Table 1 below in the specification.

The term "protein splicing" generally refers to the process by which the internal region (intein) of a precursor protein is excised and the flanking regions (exteins) of the protein are joined to form a mature protein. The intein unit may comprise the necessary components required for catalyzing protein splicing and generally comprises an endonuclease domain involved in intein mobility. The resulting proteins may be joined together but are not expressed as separate proteins. Protein splicing can also occur spontaneously with split inteins expressed on separate polypeptides to form a single intein, which then undergoes a protein splicing process to form an isolated protein, thereby occurring in a trans manner.

As used herein, the term "N-terminal of intein" refers to any such intein that comprises an N-terminal amino acid sequence that functions in a trans-splicing reaction, i.e., is capable of associating with a functional split intein C-fragment to form a complete intein sequence, which is capable of excising itself from the host protein, catalyzing the joining of exteins or flanking sequences via peptide bonds, or catalyzing a "N-terminal cleavage" when associating with a split intein C-fragment, i.e., a nucleophilic attack on the peptide bond between the extein and the N-terminus of the split intein N-fragment, resulting in cleavage of said peptide bond.

Herein, the term "C-terminal of intein" refers to any such intein that comprises a C-terminal amino acid sequence that functions in a trans-splicing reaction, i.e., is capable of associating with a functional split intein N-fragment upon association to form a complete intein, the complete intein sequence being capable of excising itself from the host protein, catalyzing the joining of exteins or flanking sequences via peptide bonds, or catalyzing a "C-terminal cleavage" when associating with a split N-intein, i.e., a nucleophilic attack on the peptide bond between the extein and the C-terminus of the C-fragment of the split intein, resulting in cleavage of said peptide bond.

The N-terminal of intein and the C-terminal of intein may be fused to the N-terminal truncated form of the ABCA4 protein and the C-terminal truncated form of the ABCA4 protein, respectively, to ligate the N-terminal truncated form of the ABCA4 protein and the C-terminal truncated form of the ABCA4 protein. For example, in some embodiments, the N-terminal of intein is fused to the C-terminus of the N-terminal portion of the split ABCA4 protein, i.e., forming a structure of N-[N-terminal truncated form of the ABCA4 protein]-[N-terminal of intein]-C. In some embodiments, the C-terminal of intein is fused to the N-terminus of the C-terminal truncated form of the ABCA4 protein, i.e., forming a structure of N-[C-terminal of intein]-[C-terminal truncated form of the ABCA4 protein]-C. The mechanism of intein-mediated protein splicing for ligating the proteins to which the intein is fused (e.g., the split ABCA4 protein) is known in the art, for example, as described in Shah et al., Chem Sci. 2014; 5(1):446-461, which is incorporated herein by reference.

In one embodiment, the "N-terminal of intein" and "C-terminal of intein" in the present invention are a homologous pair, i.e., from the same intein (see Shah et al. Journal of the American Chemical Society 2012, https://doi.org/10.1021/ja303226x). It should be understood that whether the "N-terminal of intein" and "C-terminal of intein" in the present invention are from the same intein or not, they should be capable of associating or reconstituting into a single fragment so as to carry out the trans-splicing reaction of the intein. In the present invention, preferably the "N-terminal of intein" and "C-terminal of intein" are the combinations of the following N-terminal and C-terminal of inteins, respectively: the N-terminal of GP41-1, the C-terminal of GP41-1; the N-terminal of DnaE, the C-terminal of DnaE; the N-terminal of GP41-8, the C-terminal of GP41-8; the N-terminal of NrdJ-1, the C-terminal of NrdJ-1; the N-terminal of IMPDH-1, the C-terminal of IMPDH-1; the N-terminal of SspGyrB, the C-terminal of SspGyrB; the N-terminal of Mja-KlbA, the C-terminal of Mja-KlbA; the N-terminal of NpuSsp, the C-terminal of NpuSsp; or the N-terminal of Rma, the C-terminal of Rma. In one embodiment, the sequences of the N-terminal and C-terminal of the above-mentioned inteins according to the present invention are shown in Table 1 below.

Herein, the term "degradation signal" refers to such a polypeptide that, when directly or indirectly fused to the N-terminal or C-terminal of intein of the present invention through a linker, can induce the degradation of the N-terminal or C-terminal fragment of intein. Nonlimiting examples of degradation signals comprise polypeptides related to DHFR (dihydrofolate reductase), FKBP (FK506-binding protein), FRB (FKBP-rapamycin-binding protein), and PDE5 (phosphodiesterase type 5), as well as degradation signals of the endoplasmic reticulum-associated degradation (ERAD) pathway.

In one embodiment, the N-terminal or C-terminal fragment of the intein in the first expression cassette can be directly or indirectly linked to a degradation signal at its C-terminus to promote the degradation of the intein formed after the intein splicing releases the ABCA4 protein. It should be noted that when a degradation signal is used, it will not have an adverse effect on the splicing efficiency or have a negative impact on the expressed intact ABCA4 protein.

In a preferred embodiment, the first expression cassette and the second expression cassette each sequentially comprises, starting from the 5'-end:
1) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of GP41-1 (SEQ ID NO: 17)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of the intein (the C-terminal of GP41-1 (SEQ ID NO: 18)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
2) a promoter (e.g., OPEFS promoter (SEQ ID NO: 47)), an (optimized) nucleotide coding sequence of the N-terminal truncated form of ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (N-terminal of DnaE (SEQ ID NO: 15)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of DnaE (SEQ ID NO: 16)), an (optimized) nucleotide coding sequence of the C-terminal truncated form of ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
3) a promoter (e.g., OPEFS promoter (SEQ ID NO: 47)), an (optimized) nucleotide coding sequence of the N-terminal truncated form of ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (N-terminal of GP41-8 (SEQ ID NO: 19)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of GP41-8 (SEQ ID NO: 20)), an (optimized) nucleotide coding sequence of the C-terminal truncated form of ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
4) a promoter (e.g., OPEFS promoter (SEQ ID NO: 47)), an (optimized) nucleotide coding sequence of the N-terminal truncated form of ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (N-terminal of NrdJ-1 (SEQ ID NO: 21)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of NrdJ-1 (SEQ ID NO: 22)), an (optimized) nucleotide coding sequence of the C-terminal truncated form of ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
5) a promoter (e.g., OPEFS promoter (SEQ ID NO: 47)), an (optimized) nucleotide coding sequence of the N-terminal truncated form of ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (N-terminal of IMPDH-1 (SEQ ID NO: 23)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of IMPDH-1 (SEQ ID NO: 24)), an (optimized) nucleotide coding sequence of the C-terminal truncated form of ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
6) a promoter (e.g., OPEFS promoter (SEQ ID NO: 47)), an (optimized) nucleotide coding sequence of the N-terminal truncated form of ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (N-terminal of SspGyrB (SEQ ID NO: 25)), a nucleotide coding sequence of the first tag (e.g., HA tag (SEQ ID NO: 12)), and a terminator (e.g., BGH terminator (SEQ ID NO: 51)), and
   a promoter (e.g., the OPEFS promoter (SEQ ID NO: 47)), a nucleotide coding sequence of the C-terminal of intein (the C-terminal of SspGyrB (SEQ ID NO: 26)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
7) a promoter (e.g., the OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of Mja-KlbA (SEQ ID NO: 27)), a nucleotide coding sequence of the first tag (e.g., the HA tag (SEQ ID NO: 12)), and a terminator (e.g., the BGH terminator (SEQ ID NO: 51)), and
   a promoter (e.g., the OPEFS promoter (SEQ ID NO: 47)), a nucleotide coding sequence of the C-terminal of intein (the C-terminal of Mja-KlbA (SEQ ID NO: 28)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
8) a promoter (e.g., the OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (N-terminal of NpuSsp (SEQ ID NO: 29)), a nucleotide coding sequence of the first tag (e.g., the HA tag (SEQ ID NO: 12)), and a terminator (e.g., the BGH terminator (SEQ ID NO: 51)), and
   a promoter (e.g., the OPEFS promoter (SEQ ID NO: 47)), a nucleotide coding sequence of the C-terminal of intein (the C-terminal of NpuSsp (SEQ ID NO: 30)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
9) a promoter (e.g., OPEFS promoter (SEQ ID NO: 47)), an (optimized) nucleotide coding sequence of the N-terminal truncated form of the ABCA4 protein (truncation site: amino acid position 1139 from the N-terminus, i.e., amino acids 1-1139 from the N-terminus) (the corresponding nucleotide coding sequence in SEQ ID NO: 3), a nucleotide coding sequence of the N-terminal of intein (N-terminal of GP41-1 (SEQ ID NO: 17)), a nucleotide coding sequence of the first tag (e.g., HA tag (SEQ ID NO: 12)), and a terminator (e.g., BGH terminator (SEQ ID NO: 51)), and
   a promoter (e.g., the OPEFS promoter (SEQ ID NO: 47)), a nucleotide coding sequence of the C-terminal of intein (the C-terminal of GP41-1 (SEQ ID NO: 18)), an (optimized) nucleotide coding sequence of the C-terminal truncated form of the ABCA4 protein (truncation site: amino acid position 1140 from the N-terminus, i.e., amino acids 1140-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
10) a promoter (e.g., OPEFS promoter (SEQ ID NO: 47)), an (optimized) nucleotide coding sequence of the N-terminal truncated form of ABCA4 protein (truncation site: amino acid position 1150 from the N-terminus, i.e., amino acids 1-1149 from the N-terminus) (the corresponding nucleotide coding sequence in SEQ ID NO: 3), a nucleotide coding sequence of the N-terminal of intein (N-terminal of GP41-1 (SEQ ID NO: 17)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of GP41-1 (SEQ ID NO: 18)), an (optimized) nucleotide coding sequence of the C-terminal truncated form of ABCA4 protein (truncation site: amino acid position 1150 from the N-terminus, i.e., amino acids 1150-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
11) a promoter (e.g., OPEFS promoter (SEQ ID NO: 47)), an (optimized) nucleotide coding sequence of the N-terminal truncated form of ABCA4 protein (truncation site: amino acid position 1188 from the N-terminus, i.e., amino acids from 1-1187 from the N-terminus) (the corresponding nucleotide coding sequence in SEQ ID NO: 3), a nucleotide coding sequence of the N-terminal of intein (N-terminal of GP41-1 (SEQ ID NO: 17)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of GP41-1 (SEQ ID NO: 18)), an (optimized) nucleotide coding sequence of the C-terminal truncated form of ABCA4 protein (truncation site: amino acid position 1188 from the N-terminus, i.e., amino acids 1188-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
12) a promoter (e.g., OPEFS promoter (SEQ ID NO: 47)), an (optimized) nucleotide coding sequence of the N-terminal truncated form of ABCA4 protein (truncation site: amino acid position 1139 from the N-terminus, i.e., amino acids 1-1139 from the N-terminus) (the corresponding nucleotide coding sequence in SEQ ID NO: 3), a nucleotide coding sequence of the N-terminal of intein (N-terminal of DnaE (SEQ ID NO: 15)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of DnaE (SEQ ID NO: 16)), an (optimized) nucleotide coding sequence of the C-terminal truncated form of ABCA4 protein (truncation site: amino acid position 1140 from the N-terminus, i.e., amino acids 1140-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
13) a promoter (e.g., OPEFS promoter (SEQ ID NO: 47)), an (optimized) nucleotide coding sequence of the N-terminal truncated form of ABCA4 protein (truncation site: amino acid position 1150 from the N-terminus, i.e., amino acids 1-1149 from the N-terminus) (the corresponding nucleotide coding sequence in SEQ ID NO: 3), a nucleotide coding sequence of the N-terminal of intein (N-terminal of DnaE (SEQ ID NO: 15)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of DnaE (SEQ ID NO: 16)), an (optimized) nucleotide coding sequence of the C-terminal truncated form of ABCA4 protein (truncation site: amino acid position 1150 from the N-terminus, i.e., amino acids 1150-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
14) a promoter (e.g., OPEFS promoter (SEQ ID NO: 47)), an (optimized) nucleotide coding sequence of the N-terminal truncated form of ABCA4 protein (truncation site: amino acid position 1188 from the N-terminus, i.e., amino acids 1-1187 from the N-terminus) (the corresponding nucleotide coding sequence in SEQ ID NO: 3), a nucleotide coding sequence of the N-terminal of intein (N-terminal of DnaE (SEQ ID NO: 15)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of DnaE (SEQ ID NO: 16)), an (optimized) nucleotide coding sequence of the C-terminal truncated form of ABCA4 protein (truncation site: amino acid position 1188 from the N-terminus, i.e., amino acids 1188-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
15) a promoter (e.g., OPEFS promoter (SEQ ID NO: 47)), an (optimized) nucleotide coding sequence of the N-terminal truncated form of ABCA4 protein (truncation site: amino acid position 1150 from the N-terminus, i.e., amino acids 1-1149 from the N-terminus) (the corresponding nucleotide coding sequence in SEQ ID NO: 3), a nucleotide coding sequence of the N-terminal of intein (N-terminal of DnaE (SEQ ID NO: 15)), a degradation signal (e.g., ecDHFR (SEQ ID NO: 14)), a nucleotide coding sequence of the first tag (e.g., HA tag (SEQ ID NO: 12)), and a terminator (e.g., BGH terminator (SEQ ID NO: 51)), and
   a promoter (e.g., OPEFS promoter (SEQ ID NO: 47)), a nucleotide coding sequence of the C-terminal of intein (C-terminal of DnaE (SEQ ID NO: 16)), an (optimized) nucleotide coding sequence of the C-terminal truncated form of ABCA4 protein (truncation site: amino acid position 1150 from the N-terminus, i.e., amino acids 1150-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., BGH terminator (SEQ ID NO: 51));
16) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of GP41-1 (SEQ ID NO: 17)), and a terminator (e.g., SV40 terminator (SEQ ID NO: 50)), and
   a promoter (e.g., OPEFS promoter (SEQ ID NO: 47)), a nucleotide coding sequence of the C-terminal of intein (the C-terminal of GP41-1 (SEQ ID NO: 18)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), and a terminator (e.g., SV40 terminator (SEQ ID NO: 50));
17) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of GP41-1 (SEQ ID NO: 17)), and a terminator (e.g., WPRE-SV40 terminator (SEQ ID NO: 56)), and
   a promoter (e.g., OPEFS promoter (SEQ ID NO: 47)), a nucleotide coding sequence of the C-terminal of intein (the C-terminal of GP41-1 (SEQ ID NO: 18)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), and a terminator (e.g., WPRE-SV40 terminator (SEQ ID NO: 56);
18) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of GP41- (SEQ ID NO: 17)), and a terminator (e.g., WPRE-3 terminator (SEQ ID NO: 52)), and
   a promoter (e.g., OPEFS promoter (SEQ ID NO: 47)), a nucleotide coding sequence of the C-terminal of intein (the C-terminal of GP41-1 (SEQ ID NO: 18)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), and a terminator (e.g., WPRE-3 terminator (SEQ ID NO: 52));
19) a promoter (e.g., hGRK1 promoter (SEQ ID NO: 48)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of GP41-1 (SEQ ID NO: 17)), a nucleotide coding sequence of the first tag (e.g., HA tag (SEQ ID NO: 12)), and a terminator (e.g., SV40 terminator (SEQ ID NO: 50)), and
   a promoter (e.g., hGRK1 promoter (SEQ ID NO: 48)), a nucleotide coding sequence of the C-terminal of intein (the C-terminal of GP41-1 (SEQ ID NO: 18)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., SV40 terminator (SEQ ID NO: 50)); or
20) a promoter (e.g., smCAG promoter (SEQ ID NO: 49)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of GP41-1 (SEQ ID NO: 17)), a nucleotide coding sequence of the first tag (e.g., HA tag (SEQ ID NO: 12)), and a terminator (e.g., SV40 terminator (SEQ ID NO: 50)), and
   a promoter (e.g., the smCAG promoter (SEQ ID NO: 49)), a nucleotide coding sequence of the C-terminal of the intein (the C-terminal of GP41-1 (SEQ ID NO: 18)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the SV40 terminator (SEQ ID NO: 50)).
21) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of RMA (SEQ ID NO: 61)), a nucleotide coding sequence of the first tag (e.g., HA tag (SEQ ID NO: 12)), and a terminator (e.g., BGH terminator (SEQ ID NO: 51)), and
   a promoter (e.g., the OPEFS promoter (SEQ ID NO: 47)), a nucleotide coding sequence of the C-terminal of intein (the C-terminal of RMA (SEQ ID NO: 62)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
22) a promoter (e.g., the OPEFS promoter (SEQ ID NO: 47)), an (optimized) nucleotide coding sequence of the N-terminal truncated form of the ABCA4 protein (truncation site: amino acid position 1139 from the N-terminus, i.e., amino acids 1-1139 from the N-terminus) (the corresponding nucleotide coding sequence in SEQ ID NO: 3), a nucleotide coding sequence of the N-terminal of intein (the N-terminal of RMA (SEQ ID NO: 61)), a nucleotide coding sequence of the first tag (e.g., the HA tag (SEQ ID NO: 12)), and a terminator (e.g., the BGH terminator (SEQ ID NO: 51)), and
   a promoter (e.g., the OPEFS promoter (SEQ ID NO: 47)), the C-terminal of the intein (the C-terminal of RMA (SEQ ID NO: 62)), an (optimized) nucleotide coding sequence of the C-terminal truncated form of the ABCA4 protein (truncation site: amino acid position 1140 from the N-terminus, i.e., amino acids 1140-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
23) a promoter (e.g., the OPEFS promoter (SEQ ID NO: 47)), an (optimized) nucleotide coding sequence of the N-terminal truncated form of the ABCA4 protein (truncation site: amino acid position 1150 from the N-terminus, i.e., amino acids 1-1149 from the N-terminus) (the corresponding nucleotide coding sequence in SEQ ID NO: 3), a nucleotide coding sequence of the N-terminal of intein (the N-terminal of RMA (SEQ ID NO: 61)), a nucleotide coding sequence of the first tag (e.g., the HA tag (SEQ ID NO: 12)), and a terminator (e.g., the BGH terminator (SEQ ID NO: 51)), and
   a promoter (e.g., the OPEFS promoter (SEQ ID NO: 47)), a nucleotide coding sequence of the C-terminal of the intein (the C-terminal of RMA (SEQ ID NO: 62)), an (optimized) nucleotide coding sequence of the C-terminal truncated form of the ABCA4 protein (truncation site: amino acid position 1150 from the N-terminus, i.e., amino acids 1150-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
24) a promoter (e.g., the OPEFS promoter (SEQ ID NO: 47)), an (optimized) nucleotide coding sequence of the N-terminal truncated form of the ABCA4 protein (truncation site: amino acid position 1188 from the N-terminus, i.e., amino acids 1-1187 from the N-terminus) (the corresponding nucleotide coding sequence in SEQ ID NO: 3), a nucleotide coding sequence of the N-terminal of intein (the N-terminal of RMA (SEQ ID NO: 61)), a nucleotide coding sequence of the first tag (e.g., the HA tag (SEQ ID NO: 12)), and a terminator (e.g., the BGH terminator (SEQ ID NO: 51)), and
   a promoter (e.g., the OPEFS promoter (SEQ ID NO: 47)), the C-terminal of inteins (the C-terminal of RMA (SEQ ID NO: 62)), an (optimized) nucleotide coding sequence of the C-terminal truncated form of the ABCA4 protein (truncation site: amino acid position 1188 from the N-terminus, i.e., amino acids 1188-2273 from the N-terminus) (the corresponding nucleotide coding sequence in SEQ ID NO: 3), a nucleotide coding sequence of the second tag (e.g., the Flag tag (SEQ ID NO: 13)), and a terminator (e.g., the BGH terminator (SEQ ID NO: 51));
25) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of RMA (SEQ ID NO: 61)), and a terminator (e.g., the SV4 terminator (SEQ ID NO: 50)), and
   a promoter (e.g., the OPEFS promoter (SEQ ID NO: 47)), a nucleotide coding sequence of the C-terminal of intein (the C-terminal of RMA (SEQ ID NO: 62)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), and a terminator (e.g., the SV40 terminator (SEQ ID NO: 50));
26) a promoter (e.g., the OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of RMA (SEQ ID NO: 61)), and a terminators (e.g., the WPRE-SV40 terminator (SEQ ID NO: 56)), and
   a promoter (e.g., the OPEFS promoter (SEQ ID NO: 47)), the C-terminal of inteins (the C-terminal of RMA (SEQ ID NO: 62)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), and a terminators (e.g., the WPRE-SV40 terminator (SEQ ID NO: 56));
27) a promoter (e.g., OPEFS promoter (SEQ ID NO: 47)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of RMA (SEQ ID NO: 61)), and a terminator (e.g., WPRE-3 terminator (SEQ ID NO: 52)), and
   a promoter (e.g., OPEFS promoter (SEQ ID NO: 47)), a nucleotide coding sequence of the C-terminal of intein (the C-terminal of RMA (SEQ ID NO: 62)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), and a terminator (e.g., WPRE-3 terminator (SEQ ID NO: 52));
28) a promoter (e.g., hGRK1 promoter (SEQ ID NO: 48)), an (optimized) nucleotide coding sequence of the N-terminal truncated form of the ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of RMA (SEQ ID NO: 61)), a nucleotide coding sequence of the first tag (e.g., HA tag (SEQ ID NO: 12)), and a terminator (e.g., SV40 terminator (SEQ ID NO: 50)), and
   a promoter (e.g., hGRK1 promoter (SEQ ID NO: 48)), a nucleotide coding sequence of the C-terminal of intein (the C-terminal of RMA (SEQ ID NO: 62)), an (optimized) nucleotide coding sequence of the C-terminal truncated form of the ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., SV40 terminator (SEQ ID NO: 50)); or
29) a promoter (e.g., smCAG promoter (SEQ ID NO: 49)), an (optimized) nucleotide coding sequence of the N-terminal truncated form of ABCA4 protein (truncation site: amino acid position 1224 from the N-terminus, i.e., amino acids 1-1223 from the N-terminus) (the corresponding nucleotide coding sequence in SEQ ID NO: 3), a nucleotide coding sequence of the N-terminal of intein (the N-terminal of RMA (SEQ ID NO: 61)), a nucleotide coding sequence of the first tag (e.g., HA tag (SEQ ID NO: 12)), and a terminator (e.g., SV40 terminator (SEQ ID NO: 50)), and
   a promoter (e.g., smCAG promoter (SEQ ID NO: 49)), a nucleotide coding sequence of the C-terminal of intein (the C-terminal of RMA (SEQ ID NO: 62)), an (optimized) nucleotide coding sequence of the C-terminal truncated form of ABCA4 protein (truncation site: amino acid position 1224 from the N-terminus, i.e., amino acids 1224-2273 from the N-terminus) (the corresponding nucleotide coding sequence in SEQ ID NO: 3), a nucleotide coding sequence of the second tag (e.g., Flag tag (SEQ ID NO: 13)), and a terminator (e.g., SV40 terminator (SEQ ID NO: 50)).

The term "liposome" generally refers to a lipid structure formed by amphiphilic vesicle-forming lipids. The liposome can be a closed vesicle composed of a single or multiple lipid bilayers with an internal aqueous phase. For example, in a broader sense, a liposome can refer to a lipid composite particle. The liposome can even comprise a complex whose aqueous phase has not been definitively identified. The lipid complex comprises at least one type of lipid and may additionally comprise a hydrophilic polymer, polysaccharide, amino acid, etc. The lipid complex can refer to a particle formed by these components via covalent or non-covalent bonds.

The term "exosome" generally refers to extracellular secretory cells or membrane vesicles having a membrane structure composed of a lipid bilayer present in cells. The exosomes may comprise membrane bodies having an average diameter ranging from about 10 nm to about 2,000 nm. The exosomes may comprise microvesicles. Microvesicles are also known as circulating microvesicles or microparticles and are fragments of the plasma membrane with an approximate diameter range of from 100 nm to 1000 nm shed from almost all cell types. The exosomes can also comprise smaller intracellularly generated extracellular vesicles that are formed by inward budding of the limiting membrane of multivesicular bodies (MVBs), and when the MVBs fuse with the plasma membrane, it results in their secretion and deposition in body fluids (such as blood, urine). Exosomes may comprise a complex mixture of microRNA (miR), mRNA, and proteins, which reflect the transcriptional and translational state of the producing cells.

The term "AAV" generally refers to the abbreviation of adeno-associated virus and may be used to refer to the virus itself or its derivatives. The AAV may comprise AAV type 1 (AAV-1 or AAV1), AAV type 2 (AAV-2 or AAV2), AAV type 3 (AAV-3 or AAV3), AAV type 4 (AAV-4 or AAV4), AAV type 5 (AAV-5 or AAV5), AAV type 6 (AAV-6 or AAV6), AAV type 7 (AAV-7 or AAV7), AAV type 8 (AAV-8 or AAV8), AAV type 9 (AAV-9 or AAV9), avian AAV, bovine AAV, canine AAV, equine AAV, primate AAV, non-primate AAV, and ovine AAV, etc.

In a preferred embodiment, both the first expression cassette and the second expression cassette of the present invention are AAV virus packaging plasmids, wherein the first and second expression cassettes comprise AAV 5' inverted terminal repeat (5'-ITR) sequences at the 5' end of the promoter sequence and AAV 3' inverted terminal repeat (3'-ITR) sequences at the 3' end of the terminator.

The term "kit" generally refers to a package comprising one or more active ingredients in one or more suitable containers.

The term "diseases caused by ABCA4 mutations" generally refers to diseases associated with ABCA4 gene mutations, such as rare ophthalmic diseases.

The term "inherited retinal disease" generally refers to inherited retinal dystrophies or inherited retinal diseases (IRDs), which are rare ophthalmic diseases that result in the loss and/or progressive degeneration of the patient's retinal function due to genetic defects. The inherited retinal diseases often show a reversible or irreversible visual impairment in early childhood or adolescence. IRDs are refractory ophthalmic diseases, mostly manifested as abnormal protein-level metabolism, and can be treated by regulating genes, transcription and translation in the process of genetic information transmission. The inherited retinal diseases may comprise autosomal recessive retinitis pigmentosa (RP, MERTK mutation), choroideremia (CHM mutation), juvenile macular degeneration (ABCA4 mutation), Usher syndrome subtype 1B (Myo7a mutation), X-linked congenital retinoschisis (RS1 mutation), mitochondrially related Leber hereditary optic neuropathy (ND4 mutation), achromatopsia (CNGA3 mutation and CNGB3 mutation), X-linked RP (RPGR mutation), etc.

The term "inherited macular degeneration disease" generally refers to a relatively rare familial inherited disease. The inherited macular degeneration disease may comprise Stargardt disease. Stargardt disease is an autosomal recessive genetic disease that primarily affects the retinal pigment epithelium layer. Sporadic cases are relatively rare and occur more frequently in children of consanguineous marriages. Patients have macular atrophy lesions combined with retinal yellow spot deposits. Currently, there is no approved treatment plan, and Stargardt disease can cause vision loss in hundreds of thousands of people worldwide every year. The inherited macular degeneration disease can also comprise Best disease, whose lesions mainly involve the macular area of both eyes and generally do not spread to the surrounding retina. The inherited macular degeneration disease may occur at any age.

The term "age-related macular degeneration" usually refers to age-related macular degeneration (AMD), which is one of the important causes of blindness in people over 50 years old. AMD can be manifested as a progressive and irreversible loss of central vision, mainly involving the macula, retinal pigment epithelium, choroid and other parts. Clinically, early AMD is mainly characterized by the deposition of subretinal drusen, while late AMD is divided into two types: dry and wet. Dry AMD is also known as atrophic AMD, and its main pathological changes are geographic atrophy and detachment of the RPE layer, with a slow and progressive loss of vision in the affected eye; and wet AMD is also known as neovascular or exudative AMD, and its main pathological changes are choroidal neovascularization (CNV) and secondary fluid accumulation and hemorrhage. The pathogenesis of AMD is not yet clear and may involve RPE damage, mitochondrial dysfunction, oxidative stress, inflammation, activation of the complement pathway, etc. It is generally believed that the occurrence of AMD is the result of the combined action of genetic and environmental factors.

The term "retinitis pigmentosa" usually refers to Retinitis Pigmentosa (RP), which is a group of hereditary eye diseases characterized by the progressive degeneration of retinal photoreceptors. The retinitis pigmentosa may comprise progressive retinal (a transparent photoreceptive membrane on the inner surface of the posterior part of the eyeball) degenerative diseases, which can eventually lead to moderate to severe visual loss. Retinal pigment lesions are mostly hereditary. The clinical features of the retinitis pigmentosa may comprise early night blindness, concentric narrowing of the visual field, and finally a tubular visual field, bilateral blindness or near blindness, accounting for a considerable proportion among blind eye diseases. The retinitis pigmentosa can belong to hereditary rod-cone dystrophic diseases.

The term "cone-rod dystrophy" usually refers to cone-rod dystrophy (CRD), which belongs to hereditary macular degeneration diseases. The cone-rod dystrophy may comprise a group of clinical and genetic syndromes such as progressive visual loss, photophobia, nystagmus, etc., mainly involving cone cells, and in severe cases, rod cells can also be involved. The inheritance pattern is mainly autosomal dominant, recessive, and X-linked inheritance.

The term "N-retinylidene-phosphatidylethanolamine (NRPE)" usually refers to the physiological lipid substrate of ABCA4, which is sandwiched between two TMDs in the lumenal leaflet and is further stabilized by an extended loop from extracellular domain 1.

In the present application, the first expression cassette can exist independently from the second expression cassette.

In the present application, the first expression cassette and/or the second expression cassette may exist in a form selected from the group consisting of: isolated nucleic acid molecules, liposomes, and/or exosomes.

In the present application, the first expression cassette and/or the second expression cassette may exist in the form of a plasmid(s).

In the present application, the first expression cassette and/or the second expression cassette may be a viral vector(s).

In the present application, the first expression cassette and/or the second expression cassette may be AAV(s).

In another aspect, the present application provides a kit comprising the combination of expression cassettes described in the present application.

In the present application, the kit may comprise reagents and/or instruments for administering the combination of expression cassettes. For example, the kit may comprise reagents and/or instruments for transfecting AAV comprising the first expression cassette and/or the second expression cassette. For example, the kit may comprise reagents and/or instruments for injecting AAV comprising the first expression cassette and/or the second expression cassette. In the present application, the injection may comprise local injection.

In another aspect, the present application provides the use of the combination of expression cassettes described in the present application and/or the kit described in the present application in the preparation of a medicament for treating a disease, said disease including diseases caused by ABCA4 mutations.

In the present application, the disease may comprise hereditary retinal diseases.

In the present application, the disease may comprise hereditary macular degeneration diseases, age-related macular degeneration, retinitis pigmentosa, and/or cone-rod dystrophy.

By the method described in the present application, a N-terminal truncated form of a heterologous ABCA4 protein and a C-terminal truncated form of the heterologous ABCA4 protein can be expressed in a subject in need. Since the N-terminal truncated form of the heterologous ABCA4 protein and the C-terminal truncated form of the ABCA4 protein form a complete ABCA4 protein after intein splicing and can exert the biological function of the complete ABCA4 protein, it can be used to alleviate/treat/improve diseases caused by ABCA4 mutations.

In another aspect, the present application provides a method for expressing a heterologous ABCA4 gene, which may comprise administering to a subject in need the combination of expression cassettes described in the present application, and/or the kit described in the present application.

In another aspect, the present application provides a method for alleviating cell death caused by N-retinylidene-phosphatidylethanolamine (NRPE), which may comprise administering to a subject in need the combination of expression cassettes described in the present application, and/or the kit described in the present application.

In another aspect, the present application provides a method for treating a disease caused by ABCA4 mutation, which may comprise administering the expression cassette combination described in the present application, and/or the kit described in the present application, to a subject in need.

In the present application, the administration may comprise injection.

In the present application, the disease may comprise hereditary retinal diseases.

In the present application, the disease may comprise hereditary macular degeneration diseases, age-related macular degeneration, retinitis pigmentosa, and/or cone-rod dystrophy.

The present invention will be further illustrated in the following Examples. These Examples are for illustrative purposes only and are not intended to limit the scope of the present invention in any way.

### Example 1 Codon Optimization of ABCA4 Protein

The inventors of the present application performed codon optimization on the CDS region of the wild-type human ABCA4 protein (SEQ ID NO: 1), and the optimized codons are shown as SEQ ID NO: 3 in Table 1. The CDS region of the wild-type human ABCA4 protein and the optimized CDS region were inserted behind the EF1A promoter of the pFG12-PURO vector (the pFG12-PURO vector was modified from pFG12 (Addgene#14884)), respectively, and the EGFP CDS region of the pFG12 vector was replaced with the PURO CDS (having an amino acid sequence as shown in SEQ ID NO: 57 and an encoding nucleotide sequence as shown in SEQ ID NO: 58), and at the same time, the EF1A promoter (having a sequence as shown in SEQ ID NO: 59) was inserted in the reverse direction of the UBC promoter), and pFG12-PURO-ABCA4-WT-Flag and pFGI2-PURO-ABCA4-CO-Flag were obtained, respectively. By using PEI (23966-1, Polyscience) transfection, pFG12-PURO-ABCA4-WT-Flag and pFG12-PURO-ABCA4-CO-Flag were transfected into human HEK293A (Peking University), human retinal pigment epithelial cell ARPE19 (CRL-2302, ATCC), and mouse retinal photoreceptor 661W cell line (Peking University), respectively. After 48 hours, the cells were lysed with RIPA lysis buffer (C1053-100, Applygen Technologies Inc.), and the expression of ABCA4 protein was detected by WESTERN. The optimization results showed (see Figure 1) that compared with the unoptimized wild-type ABCA4 (WT), the optimized ABCA4 coding sequence (CO) could significantly increase the protein expression of ABCA4 in human HEK293A cells and ARPE19 cells (detected with the endogenous antibody of ABCA4 (clone 5B4, MABN2440, Merck)), but the expression was not enhanced in mouse-derived 661W cells. The internal reference protein was ACTB (β-actin, detection antibody: AC026, ABclonal).

### Example 2 Different Types of Split Inteins Lead to Different ABCA4 Protein Expression Efficiencies

The strategy for forming a complete ABCA4 protein using intein dual expression cassettes according to the present invention is shown in Figure 2. It is similar to the method of Tornabene, P., et al. (2019). "Intein-mediated protein trans-splicing expands adeno-associated virus transfer capacity in the retina." Sci Transl Med 11(492), and the main differences lie in the truncation position of ABCA4 and/or the inteins used.

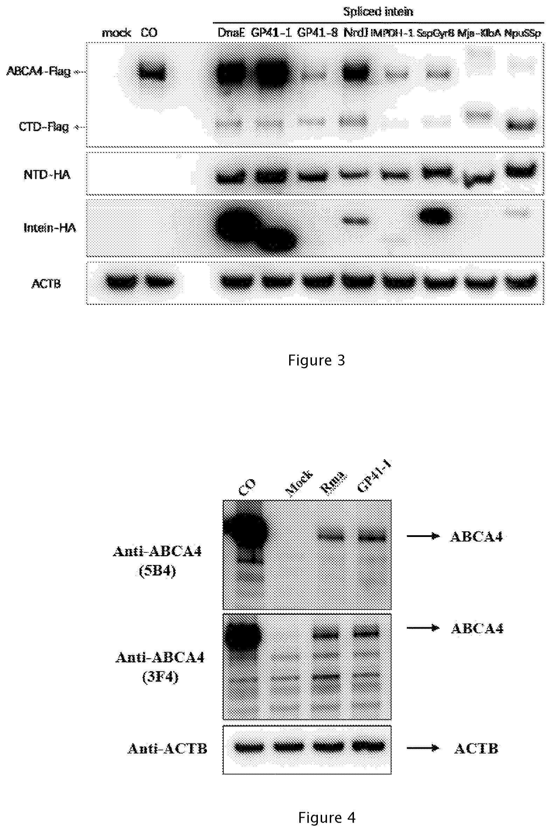

We truncated at position 1224 of the ABCA4 protein (SEQ ID NO: 1), and combined the N-terminal truncated form (NTD) and C-terminal truncated form (CTD) of ABCA4 with different types of splicing peptides, including GP41-1 (SEQ ID NO: 5), DnaE (SEQ ID NO: 4), GP41-8 (SEQ ID NO: 6), NrdJ-1 (SEQ ID NO: 7), IMPDH-1 (SEQ ID NO: 8), SspGyrB (SEQ ID NO: 9), Mja-KlbA (SEQ ID NO: 10), NpuSsp (SEQ ID NO: 11) and Rma (SEQ ID NO: 60). Using the N-terminal and C-terminal of these splicing peptides, respectively (the specific sequences are shown in Table 1 below), we inserted them into the pAV vector (pX601, Addgene) to construct the corresponding plasmids, pAV-OPEFS-NTD-intN-HA, pAV-OPEFS-intC-CTD-Flag (Note: The promoter in the plasmid is OPEFS (SEQ ID NO: 47); NTD means the plasmid contains the nucleotide coding sequence of the N-terminal truncated form of the ABCA4 protein; CTD means the plasmid comprises the nucleotide coding sequence of the C-terminal truncated form of the ABCA4 protein; intN means the plasmid comprises the nucleotide coding sequence of the N-terminal of the splicing peptide; intC means the plasmid comprises the nucleotide coding sequence of the C-terminal of the splicing peptide; HA means the plasmid comprises the nucleotide coding sequence of the HA tag (SEQ ID NO: 12); Flag means the plasmid comprises the nucleotide coding sequence of the Flag tag (SEQ ID NO: 13)). The corresponding plasmid combinations were transfected into HEK293A cells (Peking University) using PEI. The positive control CO was the plasmid pFG12-PURO-ABCA4-CO-Flag transfected. As shown in Figure 3, when the splicing peptides were DnaE, GP41-1, GP41-8, NrdJ-1, IMPDH-1 and SspGyrB, intact ABCA4 proteins with the correct molecular weight could be produced; among them, the recombination efficiencies into intact ABCA4 proteins of DnaE, GP41-1 and NrdJ were relatively high. The recombination efficiency ranking of different splicing peptides was GP41-1 > DnaE > NrdJ > GP41-8~IMPDH-1~SspGyrB. Mja-KlbA and NpuSsp could not produce intact ABCA4 proteins with a correct molecular weight. Antibodies used in WESTERN: anti-Flag, F1804, Sigma; anti-HA, 3724S, CST; anti-ACTB, AC026, ABclonal.

In addition, we further compared the splicing efficiency of GP41-1 with that of intein Rma. The results of the Western Blot experiment are shown in Figure 4. Both inteins (GP41-1 and Rma) could be effectively recombined into intact ABCA4 proteins, and the recombination efficiencies of the two inteins were comparable. Antibodies used in WESTERN: anti-ABCA4, clone 5B4; anti-ABCA4, clone 3F4; anti-ACTB, AC026, ABclonal.

### Example 3 Different Truncation Positions of ABCA4 Protein Lead to Different ABCA4 Protein Expression Efficiencies

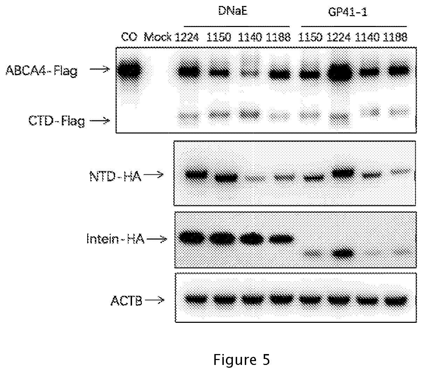

Similar to the method described in Example 2, we truncated ABCA4 at the following different amino acid positions starting from the N-terminus: 1140, 1150, 1188, and 1224, and combined the N-terminal truncated form (NTD) and C-terminal truncated form (CTD) of ABCA4 with DnaE or GP41-1 intein (splicing peptide), respectively, and the corresponding plasmid combinations were transfected into HEK293A cells (Peking University), wherein CO was the positive control transfected with plasmid pFG12-PURO-ABCA4-CO-Flag. It was found that when the truncation position was at amino acid 1224, the efficiency was relatively higher. The efficiency ranking of different truncation positions was 1224>1188>1150>1140. In addition, we found that the content of the extra small peptides intein produced by GP41-1 was less, which had a potentially higher safety in gene therapy (the results are shown in Figure 5). Antibodies used in WESTERN: anti-Flag, F1804, Sigma; anti-HA, 3724S, CST; anti-ACTB, AC026, ABclonal.

### Example 4 Dual expression cassettes comprising a degradation peptide can form a complete ABCA4 protein

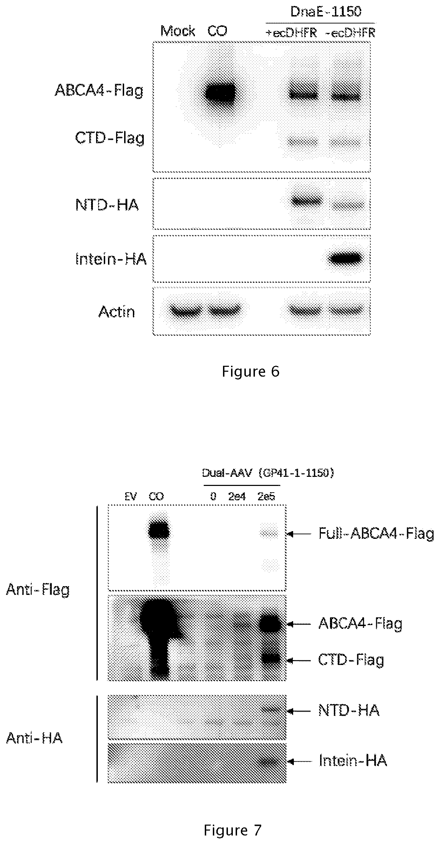

Similar to the above Examples, the N-terminal truncated form (NTD) and C-terminal truncated form (CTD) of ABCA4 obtained by truncating ABCA4 at amino acid 1150 starting from the N-terminus were combined with the N-terminal (SEQ ID NO: 15) and C-terminal (SEQ ID NO: 16) of intein DnaE, respectively, and were ligated to their respective tags to obtain two corresponding expression cassette constructs (pAV-OPEFS-NTD(1150)-int(DnaE)N-HA and pAV-OPEFS-int(DnaE)C-CTD(1150)-Flag). We inserted the coding nucleotide sequence of the expression degradation signal (degradation peptide) ecDHFR (SEQ ID NO: 14) into pAV-OPEFS-NTD(1150)-int(DnaE)N-HA to obtain the pAV-OPEFS-NTD(1150)-int(DnaE)N-ecDHFR-HA vector. The pAV-OPEFS-NTD(1150)-int(DnaE)N-HA and pAV-OPEFS-int(DnaE)C-CTD(1150)-Flag (without the degradation signal, denoted as -ecDHFR in Figure 6), or pAV-OPEFS-NTD(1150)-int(DnaE)N-ecDHFR-HA and pAV-OPEFS-int(DnaE)C-CTD(1150)-Flag (with the degradation signal, denoted as +ecDHFR in Figure 6) were co-transfected into HEK293A cells by PEI. After 48 hours, the expression of the complete ABCA4 protein was detected by WESTERN (as shown in Figure 6), wherein CO was the positive control transfected with plasmid pFG12-PURO-ABCA4-CO-Flag. The results showed that the dual expression cassettes comprising the degradation peptide could form a complete ABCA4 protein, and fewer intein was detected, indicating that the intein was partially degraded by the degradation signal. Antibodies used in WESTERN: anti-Flag, F1804, Sigma; anti-HA, 3724S, CST.

### Example 5 The expression cassettes can form a complete ABCA4 protein in 293T cells

Similar to the above Examples, N-terminal truncated form (NTD) and C-terminal truncated form (CTD) of ABCA4 obtained by truncating ABCA4 at amino acid 1150 starting from the N-terminus of ABCA4 were combined with the N-terminal and C-terminal of the intein GP41-1 (the N-terminal of GP41-1 (SEQ ID NO: 17) and the C-terminal of GP41-1 (SEQ ID NO: 18)), respectively, and were ligated to their respective tags to obtain two corresponding expression cassette constructs (pAV-OPEFS-NTD(1150)-int(GP41-1)N-HA, pAV-OPEFS-int(GP41-1)C - CTD(1150)-Flag). Using the constructed AAV8 vectors pAV-OPEFS-NTD(1150)-int(GP41-1)N-HA and pAV-OPEFS-int(GP41-1)C-CTD(1150)-Flag, the expression cassettes of the NTD and CTD (truncated at position 1150) combined with GP41-1 as described in Example 3 were packaged, respectively, and co-infected into 293T cells (3216, ATCC). The multiplicity of infection (MOI) was 0, 2E4, and 2E5, respectively. After 72 hours of infection, a complete ABCA4 protein could be formed (see the results in Figure 7), wherein CO was the positive control transfected with the plasmid pFG12-PURO-ABCA4-CO-Flag. Antibodies used in Western blotting: anti-Flag, F1804, Sigma; anti-HA, 3724S, CST.

### Example 6 The expression cassettes can successfully express a complete ABCA4 protein in ABCA4 KO mice

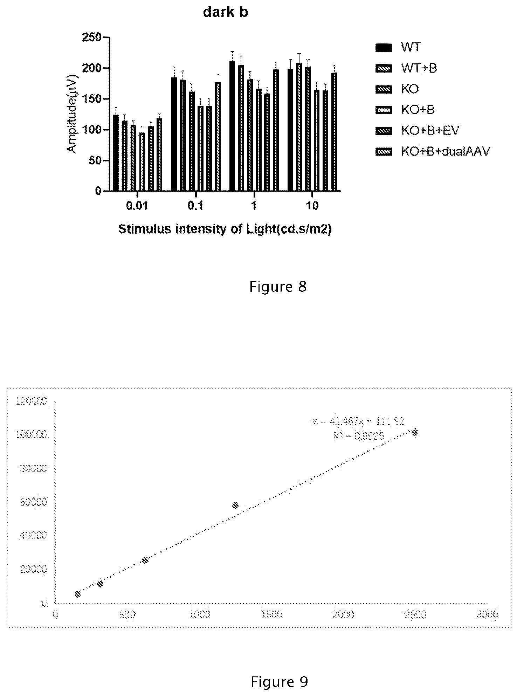

Using the AAV8 vectors pAV-OPEFS-NTD(1150)-int(GP41-1)N-HA and pAV-OPEFS-int(GP41-1)C-CTD(1150)-Flag constructed as described in the above Examples, the expression cassettes of the NTD and CTD (truncated at position 1150) combined with GP41-1 as described in Example 3 (dual-AAV (GP41-1-1150)) were packaged, respectively, and injected subretinally into 1- to 2-month-old ABCA4 KO mice (S-KO-00815, C57BL/6J, Cyagen Biosciences). Five weeks later, the mice were anesthetized and subjected to pupil dilation, and stimulated with 20000 lux of 430 nm blue light for 30 min. After 1 week of recovery, ERG was detected. The results showed that blue light stimulation could significantly reduce the ERG amplitude level of the mice, and the ERG amplitude level of ABCA4 KO mice could be restored after dual-AAV injection (see Figure 8, wherein WT represents wild-type mice; WT+B represents wild-type mice after blue light stimulation; KO represents ABCA4 KO mice; KO+B represents ABCA4 KO mice without injection after blue light stimulation; KO+B+EV represents ABCA4 KO mice injected with AAV-EV virus after blue light stimulation (wherein AAV-EV virus represents an empty AAV virus, i.e., the AAV virus group without the target gene (i.e., the ABCA4 expression cassette)); KO+B+dualAAV represents ABCA4 KO mice injected with the AAV virus expressing the dual expression cassettes according to the present invention after blue light stimulation).

Six weeks later, the animals were euthanized at the end of the detection, and the retinal tissues of the eyeballs were isolated. Part of them was used for the extraction of A2E by phenolchloroform-methanol extraction. With acetonitrile containing 0.1% TFA and water as the mobile phase, ACQUITY UPLC H-Class ultra-high performance liquid chromatography and Atlantic dC18 chromatographic column were used to detect the content of A2E in the samples (Radu, R. A., et al. (2008). "Accelerated accumulation of lipofuscin pigments in the RPE of a mouse model for ABCA4-mediated retinal dystrophies following Vitamin A supplementation." Invest Ophthalmol Vis Sci 49(9): 3821-3829.) (see Figure 9 and Table A).

**Table A**

| Sample source | Area | Concentration |
|---|---|---|
| Standard sample | 5414 | 156.25 |
| | 11316 | 312.5 |
| | 25569 | 625 |
| | 57913 | 1250 |
| | 101204 | 2500 |
| WT (Wild type) | 1813 | 41.0225 |
| KO-EV (ABCA4 KO mice injected with AAV-EV virus) | 4911 | 115.7325 |
| KO-dual-AAV (ABCA4 KO mice injected with AAV virus expressing the dual expression cassettes according to the present invention) | 2641 | 60.99018 |

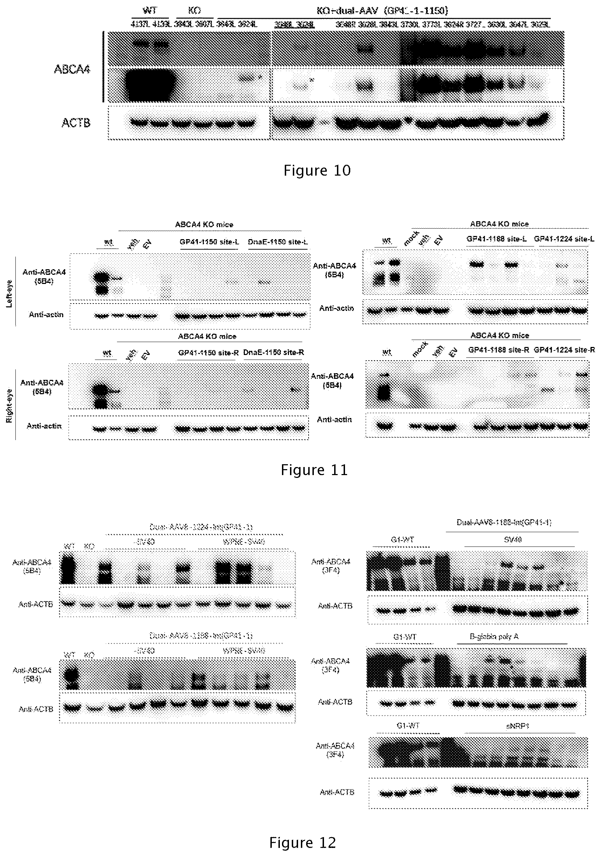

The remaining mouse retinal tissues were used for Western Blot detection. The results showed (Figure 10) that 9 out of 12 mouse retinal samples could express the complete ABCA4 protein, and the highest expression level was approximately 20% of the endogenous expression level of wild-type mice. Antibodies used in Western Blot: anti-ABCA4, clone 5B4, MABN2440, Merck; anti-ACTB, AC026, ABclonal.

### Example 7 Expression cassettes comprising different cleavage sites of ABCA4 protein can successfully express the complete ABCA4 protein in ABCA4 KO mice

According to the method of the above Examples, AAV8 vectors were used to package the following AAVs, respectively: the expression cassettes of the truncated NTD and CTD at site 1150 combined with GP41-1 (dual-AAV (GP41-1-1150)), the expression cassettes of the truncated NTD and CTD at site 1150 combined with DnaE (dual-AAV (DnaE-1150)), the expression cassettes of the truncated NTD and CTD at site 1188 combined with GP41-1 (dual-AAV (GP41-1-1188)), and the expression cassettes of the truncated NTD and CTD at site 1224 combined with GP41-1 (dual-AAV (GP41-1-1224)). Each dual-AAV combination was injected into the subretinal space of ABCA4 KO mice. After 6 weeks, the animals were euthanized, and the retinal tissues of the eyeballs were isolated for Western Blot detection. The experimental results are shown in Figure 11. The full-length ABCA4 protein could be effectively expressed in the mouse retina for each of the sites 1150, 1188, and 1224. By comparison, the cleavage efficiency at sites 1188 and 1224 was higher than that at site 1150.

### Example 8 Expression cassettes comprising different regulatory elements can successfully express the complete ABCA4 protein in ABCA4 KO mice

To further study the effect of regulatory elements on the *in vivo* expression efficiency of ABCA4, based on the above Examples, the Flag tag and HA tag were deleted from the dual-AAV vectors derived from the 1224 or 1188 cleavage site, respectively, and the original BGH terminator was replaced with the SV40 terminator or the WPRE-SV40 terminator. In addition, for that derived from the 1188 cleavage site, the dual-AAV terminator was further replaced with the B-globin-polyA terminator (SEQ ID NO: 65) or the sNRP1 terminator (SEQ ID NO: 66).

The above vectors were packaged using AAV8 vectors, respectively. Each dual-AAV combination was injected into the subretinal space of ABCA4 KO mice. After 6 weeks, the animals were euthanized, and the retinal tissues of the eyeballs were isolated for Western Blot detection. The experimental results are shown in Figure 12. Each of terminators could be used to effectively express the full-length ABCA4 protein, and WPRE could promote the expression of the complete ABCA4 protein.

### Example 9 Expression cassettes comprising different promoters can successfully express the complete ABCA4 protein in ABCA4 KO mice

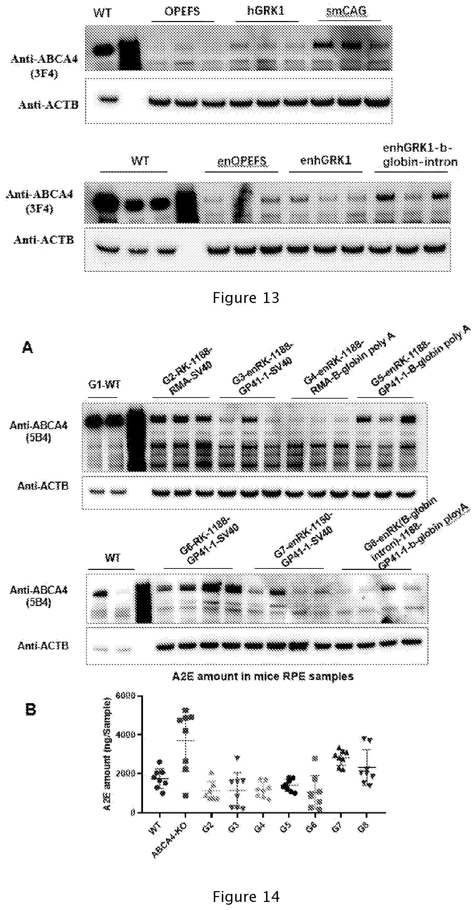

Using the vectors pAV-OPEFS-NTD(1224)-int(GP41-1)N-SV40 and pAV-OPEFS-int(GP41-1)C-CTD(1224)-SV40 constructed as described in the above Examples, the original promoter sequence EFS was replaced with the hGRK1 promoter or the smCAG promoter. In addition, add the CMV enhancer before the EFS promoter and the hGRK1 promoter to obtain the corresponding expression cassette vectors (enOPEFS promoter, SEQ ID NO: 67; enhGRK1 (enRK) promoter, SEQ ID NO: 68). At the same time, a part of the hGRK1 promoter sequence was further replaced with the b-globin-intron sequence to form a new hGRK1-b-globin-intron promoter (SEQ ID NO: 69), and then the CMV enhancer was added to obtain the enhGRK1-b-globin-intron promoter (SEQ ID NO: 70). The above vectors were packaged with the AAV8 vectors, respectively. Each dual-AAV combination was injected into the subretinal space of ABCA4 KO mice. After 6 weeks, the animals were euthanized, and the retinal tissues of the eyeballs were isolated for Western Blot detection. The experimental results are shown in Figure 13. Each of promoters could be used to effectively express the full-length ABCA4 protein in the mouse retina.

### Example 10 Reducing the length of the expression cassettes can successfully express the complete ABCA4 protein in ABCA4 KO mice

Due to the long coding amino acid sequence of the ABCA4 1224 cleavage site, the length of the ABCA4-NTD expression cassettes may be greater than the packaging capacity of the AAV vector, making it difficult to achieve an efficient production. Based on the method of the above Examples, we screened the expression cassettes based on the 1188 and 1150 cleavage sites and further verified the expression cassette combinations with reduced genomes in mice.

The element combinations of vector comprise:

| | | | | |
|---|---|---|---|---|
| 1 | hGRK1 | 1188 | Rma | SV40 |
| 2 | enRK | 1188 | GP41-1 | SV40 |
| 3 | enRK | 1188 | Rma | B-globin |
| 4 | enRK | 1188 | GP41-1 | B-globin |
| 5 | hGRK1 | 1188 | GP41-1 | SV40 |
| 6 | enRK | 1150 | GP41-1 | SV40 |
| 7 | enRK-b-globin intron (SEQ ID NO: 70) | 1188 | GP41-1 | B-globin |

The NTD and CTD expressed by the above dual expression cassettes were packaged in AAV8. Each dualAAV combination was subretinally injected into ABCA4 KO mice. After 8 weeks, the animals were euthanized, and the retinal tissues of the eyeballs were isolated for Western Blot detection. The experimental results are shown in Figure 14, A. All combinations could effectively express the full-length ABCA4 protein in the retinas of model mice. In addition, the A2E detection results are shown in Figure 14, B. After subretinal administration of the expression cassette vectors in each group, the deposition of A2E in the ABCA4 KO mouse model decreased significantly, indicating that the above expression cassettes all had a potential therapeutic effect.

### Example 11 Packaging of Expression Cassettes in Different Vectors Can Successfully Express the Complete ABCA4 Protein in ABCA4 KO Mice

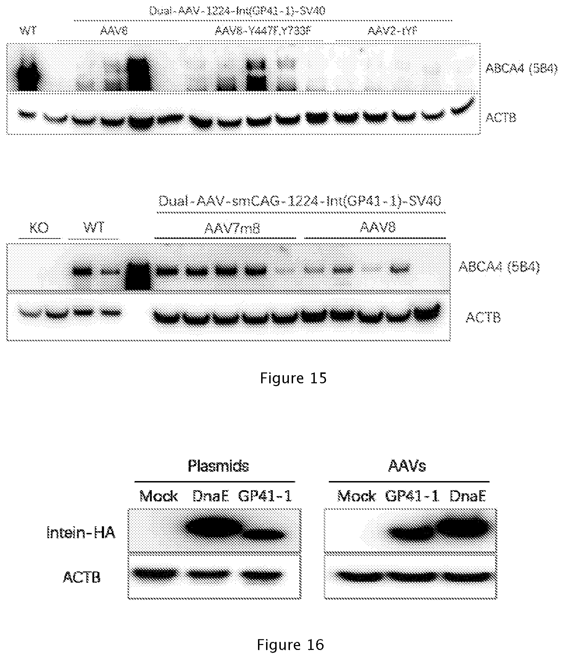

According to the method of the above Examples, AAV8, AAV8-Y447,733F vector, dual-AAVtYF or AAV2.7m8 were used to package the expression cassettes combining the truncated NTD and CTD at the 1224 site with the GP41-1 (dual-AAV8 (GP41-1-1224), dual-AAV8-Y447,733F (GP41-1-1224), dual-AAVtYF (GP41-1-1224), dual-AAV2.7m8 (GP41-1-1224)) (each virus was packaged by Guangzhou PackGene Biotechnology Co., Ltd.), and were subretinally injected into ABCA4 KO mice. After 6 weeks, the animals were euthanized, and the retinal tissues of the eyeballs were isolated for Western Blot detection. The experimental results are shown in Figure 15. The AAV8, AAV8-Y447,733F variant, and AAV2.7m8 serotypes all could effectively express the full-length ABCA4 protein in the retinas of mice, while the expression efficiency of the AAVtYF serotype was relatively low. Compared with AAV8, the expression level of the AAV7m8 serotype was stronger.

### Example 12 The Content of the Additional Small Peptides Produced by the Split Intein GP41-1 is Less

To detect whether the additional small peptides produced after the splicing of the dual AAV vector by the split peptide are toxic for retina, we constructed the plasmids pAV-OPEFS-int(GP41-1)-HA and pAV-OPEFS-int(DnaE)-HA (pX601, Addgene) with the individual GP41-1 split peptide and DnaE split peptide, respectively, and packaged them into AAV8 viruses. The following are the expression results at 48 hours after plasmid transfection of HEK293A and 72 hours after AAV infection of 293T cells (Figure 16).

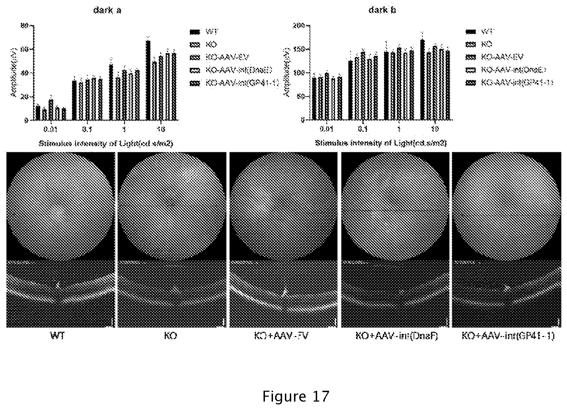

AAV8-GP41-1 and AAV8-DnaE were subretinally injected into ABCA4 KO mice, respectively. After 6 weeks, ERG (electroretinogram) (see Figure 17), fundus OCT (optical coherence tomography), and FP (fundus photograph) detections were performed. Compared with the control group, it was found that the content of the additional small peptide intein produced by GP41-1 was less, and no retina toxicity was found.

Those skilled in the art should understand that although the present invention has been specifically described with reference to the above Examples, the present invention is not limited to these particular Examples. Based on the methods and technical solutions taught by the present invention, those skilled in the art could make appropriate modifications or improvements without departing from the spirit of the present invention, and the equivalent embodiments obtained therefrom are all within the scope of the present invention.

**Table 1 List of sequences used in the specification and Examples**

| **Name** | **Sequence** | **SEQ ID NO:** |
|---|---|---|
| Human ABCA4 wild-type protein | | 1 |
| | | |
| Coding sequence of human ABCA4 wild-type protein | atgggcttcgtgagacagatacagcttttgctctggaagaactggaccctgcggaaaaggcaaaagattcgctttgtggtggaactcgtgtggcctttatctttatttctggtcttgatctggttaaggaatg ccaacccactctacagccatcatgaatgccatttccccaacaaggcgatgccctcagcaggaatgctgccgtggctccaggggatcttctgcaatgtgaacaatccctgttttcaaagccccacccca ggagaatctcctggaattgtgtcaaactataacaactccatcttggcaagggtatatcgagattttcaagaactcctcatgaatgcaccagagagccagcaccttggccgtatttggacagagctacaca tcttgtcccaattcatggacaccctccggactcacccggagagaattgcaggaagaggaatacgaataagggatatcttgaaagatgaagaaacactgacactatttctcattaaaaacatcggcctgt ctgactcagtggtctaccttctgatcaactctcaagtccgtccagagcagttcgctcatggagtcccggacctggcgctgaaggacatcgcctgcagcgaggccctcctggagcgcttcatcatcttca gccagagacgcggggcaaagacggtgcgctatgccctgtgctccctctcccagggcaccctacagtggatagaagacactctgtatgccaacgtggacttcttcaagctcttccgtgtgcttcccaca ctcctagacagccgttctcaaggtatcaatctgagatcttggggaggaatattatctgatatgtcaccaagaattcaagagtttatccatcggccgagtatgcaggacttgctgtgggtgaccaggcccct catgcagaatggtggtccagagacctttacaaagctgatgggcatcctgtctgacctcctgtgtggctaccccgagggaggtggctctcgggtgctctccttcaactggtatgaagacaataactataa ggcctttctggggattgactccacaaggaaggatcctatctattcttatgacagaagaacaacatccttttgtaatgcattgatccagagcctggagtcaaatcctttaaccaaaatcgcttggagggcgg caaagcctttgctgatgggaaaaatcctgtacactcctgattcacctgcagcacgaaggatactgaagaatgccaactcaacttttgaagaactggaacacgttaggaagttggtcaaagcctgggaa gaagtagggccccagatctggtacttctttgacaacagcacacagatgaacatgatcagagataccctggggaacccaacagtaaaagactttttgaataggcagcttggtgaagaaggtattactgct gaagccatcctaaacttcctctacaagggccctcgggaaagccaggctgacgacatggccaacttcgactggagggacatatttaacatcactgatcgcaccctccgcctggtcaatcaatacctgga gtgcttggtcctggataagtttgaaagctacaatgatgaaactcagctcacccaacgtgccctctctctactggaggaaaacatgttctgggccggagtggtattccctgacatgtatccctggaccagct ctctaccaccccacgtgaagtataagatccgaatggacatagacgtggtggagaaaaccaataagattaaagacaggtattgggattctggtcccagagctgatcccgtggaagatttccggtacatct ggggcgggtttgcctatctgcaggacatggttgaacaggggatcacaaggagccaggtgcaggcggaggctccagttggaatctacctccagcagatgccctacccctgcttcgtggacgattcttt catgatcatcctgaaccgctgtttccctatcttcatggtgctggcatggatctactctgtctccatgactgtgaagagcatcgtcttggagaaggagttgcgactgaaggagaccttgaaaaatcagggtg tctccaatgcagtgatttggtgtacctggttcctggacagcttctccatcatgtcgatgagcatcttcctcctgacgatattcatcatgcatggaagaatcctacattacagcgacccattcatcctcttcctgt | 2 |
| | tcttgttggctttctccactgccaccatcatgctgtgctttctgctcagcaccttcttctccaaggccagtctggcagcagcctgtagtggtgtcatctatttcaccctctacctgccacacatcctgtgcttcg cctggcaggaccgcatgaccgctgagctgaagaaggctgtgagcttactgtctccggtggcatttggatttggcactgagtacctggttcgctttgaagagcaaggcctggggctgcagtggagcaa catcgggaacagtcccacggaaggggacgaattcagcttcctgctgtccatgcagatgatgctccttgatgctgctgtctatggcttactcgcttggtaccttgatcaggtgtttccaggagactatggaa ccccacttccttggtactttcttctacaagagtcgtattggcttggcggtgaagggtgttcaaccagagaagaaagagccctggaaaagaccgagcccctaacagaggaaacggaggatccagagc acccagaaggaatacacgactccttctttgaacgtgagcatccagggtgggttcctggggtatgcgtgaagaatctggtaaagatttttgagccctgtggccggccagctgtggaccgtctgaacatca ccttctacgagaaccagatcaccgcattcctgggccacaatggagctgggaaaaccaccaccttgtccatcctgacgggtctgttgccaccaacctctgggactgtgctcgttgggggaagggacatt gaaaccagcctggatgcagtccggcagagccttggcatgtgtccacagcacaacatcctgttccaccacctcacggtggctgagcacatgctgttctatgcccagctgaaaggaaagtcccaggag gaggcccagctggagatggaagccatgttggaggacacaggcctccaccacaagcggaatgaagaggctcaggacctatcaggtggcatgcagagaaagctgtcggttgccattgcctttgtggg agatgccaaggtggtgattctggacgaacccacctctggggtggacccttactcgagacgctcaatctgggatctgctcctgaagtatcgctcaggcagaaccatcatcatgtccactcaccacatgg acgaggccgacctccttggggaccgcattgccatcattgcccagggaaggctctactgctcaggcaccccactcttcctgaagaactgctttggcacaggcttgtacttaaccttggtgcgcaagatga aaaacatccagagccaaaggaaaggcagtgaggggacctgcagctgctcgtctaagggtttctccaccacgtgtccagcccacgtcgatgacctaactccagaacaagtcctggatggggatgtaa atgagctgatggatgtagttctccaccatgttccagaggcaaagctggtggagtgcattggtcaagaacttatcttccttcttccaaataagaacttcaagcacagagcatatgccagccttttcagagag ctggaggagacgctggctgaccttggtctcagcagttttggaatttctgacactcccctggaagagatttttctgaaggtcacggaggattctgattcaggacctctgtttgcgggtggcgctcagcaga aaagagaaaacgtcaacccccgacacccctgcttgggtcccagagagaaggctggacagacaccccaggactccaatgtctgctccccaggggcgccggctgctcacccagagggccagcctc ccccagagccagagtgcccaggcccgcagctcaacacggggacacagctggtcctccagcatgtgcaggcgctgctggtcaagagattccaacacaccatccgcagccacaaggacttcctggc gcagatcgtgctcccggctacctttgtgtttttggctctgatgctttctattgttatccctccttttggcgaataccccgctttgacccttcacccctggatatatgggcagcagtacaccttcttcagcatggat gaaccaggcagtgagcagttcacggtacttgcagacgtcctcctgaataagccaggctttggcaaccgctgcctgaaggaagggtggcttccggagtacccctgtggcaactcaacaccctggaag | |
| | actccttctgtgtccccaaacatcacccagctgttccagaagcagaaatggacacaggtcaacccttcaccatcctgcaggtgcagcaccagggagaagctcaccatgctgccagagtgccccgag ggtgccgggggcctcccgcccccccagagaacacagcgcagcacggaaattctacaagacctgacggacaggaacatctccgacttcttggtaaaaacgtatcctgctcttataagaagcagctta aagagcaaattctgggtcaatgaacagaggtatggaggaatttccattggaggaaagctcccagtcgtccccatcacgggggaagcacttgttgggtttttaagcgaccttggccggatcatgaatgtg agcgggggccctatcactagagaggcctctaaagaaatacctgatttccttaaacatctagaaactgaagacaacattaaggtgtggtttaataacaaaggctggcatgccctggtcagctttctcaatgt ggcccacaacgccatcttacgggccagcctgcctaaggacaggagccccgaggagtatggaatcaccgtcattagccaacccctgaacctgaccaaggagcagctctcagagattacagtgctga ccacttcagtggatgctgtggttgccatctgcgtgattttctccatgtccttcgtcccagccagctttgtcctttatttgatccaggagcgggtgaacaaatccaagcacctccagtttatcagtggagtgag ccccaccacctactgggtgaccaacttcctctgggacatcatgaattattccgtgagtgctgggctggtggtgggcatcttcatcgggtttcagaagaaagcctacacttctccagaaaaccttcctgcc cttgtggcactgctcctgctgtatggatgggcggtcattcccatgatgtacccagcatccttcctgtttgatgtccccagcacagcctatgtggctttatcttgtgctaatctgttcatcggcatcaacagcag tgctattaccttcatcttggaattatttgagaataaccggacgctgctcaggttcaacgccgtgctgaggaagctgctcattgtcttcccccacttctgcctgggccggggcctcattgaccttgcactgag ccaggctgtgacagatgtctatgcccggtttggtgaggagcactctgcaaatccgttccactgggacctgattgggaagaacctgtttgccatggtggtggaaggggtggtgtacttcctcctgaccct gctggtccagcgccacttcttcctctcccaatggattgccgagcccactaaggagcccattgttgatgaagatgatgatgtggctgaagaaagacaaagaattattactggtggaaataaaactgacatc ttaaggctacatgaactaaccaagatttatccaggcacctccagcccagcagtggacaggctgtgtgtcggagttcgccctggagagtgctttggcctcctgggagtgaatggtgccggcaaaacaa ccacattcaagatgctcactggggacaccacagtgacctcaggggatgccaccgtagcaggcaagagtattttaaccaatatttctgaagtccatcaaaatatgggctactgtcctcagtttgatgcaatt gatgagctgctcacaggacgagaacatctttacctttatgcccggcttcgaggtgtaccagcagaagaaatcgaaaaggttgcaaactggagtattaagagcctgggcctgactgtctacgccgactg cctggctggcacgtacagtgggggcaacaagcggaaactctccacagccatcgcactcattggctgcccaccgctggtgctgctggatgagcccaccacagggatggacccccaggcacgccgc atgctgtggaacgtcatcgtgagcatcatcagagaagggagggctgtggtcctcacatcccacagcatggaagaatgtgaggcactgtgtacccggctggccatcatggtaaagggcgcctttcgat gtatgggcaccattcagcatctcaagtccaaatttggagatggctatatcgtcacaatgaagatcaaatccccgaaggacgacctgcttcctgacctgaaccctgtggagcagttcttccaggggaactt | |
| | cccaggcagtgtgcagagggagaggcactacaacatgctccagttccaggtctcctcctccctggcgaggatcttccagctcctctcccacaaggacagcctgctcatcgaggagtactca gtcacacagaccacactggaccaggtgtttgtaaattttgctaaacagcagactgaaagtcatgacctccctctgcaccctcgagctgctggagccagtcgacaagcccaggactga | |
| Optimized codon for human ABCA4 protein | ATGGGATTCGTGCGGCAGATCCAGCTTCTGCTCTGGAAGAATTGGACCCTGCGTAAACGGCAGAAGATTAGATTTGTGG TGGAGCTGGTGTGGCCTCTGAGCCTGTTCTTGGTGCTGATCTGGCTGCGAAACGCCAACCCTCTGTACTCCCACCATGAG TGTCACTTCCCTAACAAGGCCATGCCTTCTGCTGGCATGCTGCCTTGGCTGCAGGGCATCTTCTGCAACGTGAACAACCC CTGCTTCCAGAGCCCCACCCCTGGAGAGTCTCCCGGCATCGTCAGTAACTACAACAACTCCATCCTCGCCAGAGTGTAC AGAGATTTCCAGGAGCTGCTGATGAACGCCCCTGAGTCTCAACACCTGGGGAGAATCTGGACCGAGCTCCATATACTGT CACAGTTCATGGATACCCTACGGACCCACCCCGAACGGATCGCCGGCAGAGGCATTAGAATCCGGGATATCCTTAAGGA CGAAGAGACACTGACTCTTTTTCTGATTAAGAATATCGGATTGTCTGATTCGGTGGTGTACCTGCTGATCAACAGCCAGG TGAGACCTGAGCAGTTCGCCCACGGCGTGCCTGACTTAGCGCTGAAGGACATTGCCTGCTCTGAGGCCTTGCTGGAAAG ATTCATCATCTTCTCTCAGCGGCGGGGGGCCAAGACAGTTCGGTACGCCCTGTGCAGCCTGAGCCAGGGCACCTTGCAG TGGATCGAAGACACCCTGTATGCTAACGTGGACTTCTTTAAACTGTTTAGAGTGTTGCCAACCTTACTGGACAGCCGGA GCCAGGGCATCAACCTGAGATCCTGGGGTGGAATCCTGAGCGATATGAGCCCCAGAATCCAGGAGTTCATCCACCGGCC TAGCATGCAGGACTTGCTGTGGGTGACTCGTCCCCTGATGCAAAACGGAGGACCAGAAACATTTACAAAGCTTATGGGC ATCCTGAGCGACCTCTTGTGCGGCTACCCTGAGGGCGGCGGCAGCCGGGTGTTAAGCTTTAACTGGTACGAGGACAACA ACTACAAGGCCTTCCTTGGTATCGATAGCACACGAAAGGACCCAATCTACAGCTACGACCGTAGAACAACCAGTTTCTG | 3 |
| | CAACGCCCTGATCCAGTCCCTTGAATCTAATCCTTTGACAAAGATCGCTTGGCGGGCCGCCAAGCCTCTGCTGATGGGC AAGATCCTGTACACCCCTGACAGCCCTGCCGCCAGAAGAATCCTAAAGAATGCCAACAGCACTTTCGAGGAACTCGAAC ACGTGCGGAAGCTGGTCAAAGCCTGGGAGGAGGTGGGCCCTCAAATCTGGTACTTCTTCGATAACTCCACCCAGATGAA CATGATCAGAGACACCTTGGGAAATCCGACCGTGAAGGACTTCCTGAATAGACAGCTAGGTGAGGAGGGAATCACCGC CGAGGCCATCCTGAACTTTCTGTACAAAGGCCCTCGGGAGTCCCAGGCTGATGACATGGCCAATTTTGACTGGAGAGAC ATATTCAACATCACAGACCGTACCCTTAGACTGGTGAACCAGTACCTGGAATGCTTAGTCCTGGATAAGTTCGAAAGCT ACAACGACGAGACCCAGCTGACCCAACGGGCACTGTCTCTACTGGAGGAGAATATGTTCTGGGCCGGTGTGGTGTTCCC TGACATGTACCCCTGGACAAGCAGCCTGCCACCTCACGTGAAATACAAGATCCGGATGGACATTGACGTGGTGGAAAA GACAAACAAGATCAAGGACAGATACTGGGACAGCGGACCTAGGGCCGATCCTGTGGAGGATTTCCGGTACATCTGGGG TGGCTTCGCTTACCTGCAGGACATGGTGGAACAGGGCATCACGAGAAGCCAGGTGCAGGCTGAGGCCCCTGTGGGAAT CTACCTCCAACAGATGCCCTACCCCTGCTTCGTGGACGACAGCTTCATGATCATCCTGAATAGATGTTTCCCAATCTTCA TGGTGCTGGCCTGGATCTACAGCGTGTCCATGACTGTGAAGTCCATCGTGCTGGAGAAGGAGCTGCGGCTAAAAGAGAC TCTGAAGAATCAGGGCGTGAGCAACGCCGTAATCTGGTGCACCTGGTTCCTGGACTCCTTCTCCATCATGAGCATGAGC ATCTTCCTACTGACAATCTTTATCATGCACGGCAGAATTCTGCACTACAGCGACCCTTTCATCCTGTTTCTTTTTTTGCTG GCTTTCAGCACCGCCACCATCATGCTGTGCTTCCTGTTAAGCACCTTCTTCTCAAAGGCCAGCCTAGCTGCAGCATGCTC CGGCGTCATCTACTTTACTTTATACCTGCCCCACATCCTGTGTTTCGCCTGGCAGGACAGAATGACAGCCGAGCTGAAAA AGGCCGTATCCCTGTTGAGCCCCGTGGCCTTCGGGTTTGGAACCGAGTACCTGGTACGGTTCGAGGAACAAGGCCTGGG | |
| | CCTGCAGTGGTCCAACATCGGCAACTCGCCGACCGAGGGCGACGAATTCAGCTTCCTGCTGTCCATGCAGATGATGCTG CTGGATGCCGCAGTGTATGGCCTCCTGGCTTGGTACTTGGACCAGGTGTTCCCCGGCGACTACGGGACTCCACTGCCATG GTACTTTCTGCTGCAGGAGAGCTACTGGCTGGGCGGCGAGGGCTGCAGCACCAGAGAGGAAAGAGCCCTGGAGAAGAC AGAGCCTCTGACTGAAGAAACCGAAGACCCTGAGCACCCTGAGGGGATACACGACTCTTTTTTCGAGAGGGAACATCCT GGATGGGTCCCTGGCGTTTGCGTGAAGAACCTGGTGAAGATCTTCGAGCCTTGTGGCAGGCCAGCCGTGGATAGACTGA ATATCACCTTCTACGAGAACCAGATCACCGCCTTCCTGGGCCACAACGGCGCGGGAAAGACCACAACTCTGAGCATCCT GACCGGACTGCTGCCTCCAACTTCCGGCACCGTGCTCGTCGGCGGCAGGGACATCGAGACCAGTCTGGACGCCGTGAGA CAGAGCCTCGGCATGTGCCCTCAGCACAATATCCTGTTTCACCACTTAACCGTTGCAGAACACATGCTGTTCTACGCCCA GTTAAAAGGCAAGTCGCAGGAGGAGGCCCAGCTGGAGATGGAAGCAATGCTCGAAGATACAGGCCTGCACCATAAGA GAAACGAAGAGGCCCAGGATCTCTCCGGCGGCATGCAGAGAAAACTGAGCGTAGCCATCGCCTTCGTGGGAGATGCAA AGGTGGTAATCCTAGATGAGCCTACATCTGGGGTGGACCCGTACAGCCGGCGCAGTATTTGGGATTTACTGCTGAAGTA CAGAAGCGGTAGAACCATCATCATGTCTACCCACCACATGGACGAGGCTGATCTGCTGGGAGATCGGATCGCCATAATC GCCCAGGGAAGACTGTATTGCAGCGGCACACCACTGTTCCTCAAAAACTGCTTTGGGACGGGACTGTACCTGACGCTGG TCCGGAAAATGAAGAACATCCAGTCCCAGAGAAAGGGCTCTGAAGGGACCTGTTCTTGCAGCTCTAAGGGCTTCAGTAC CACCTGCCCTGCCCACGTGGACGACCTGACTCCCGAACAGGTGCTGGACGGCGATGTGAACGAGCTGATGGACGTGGTG CTGCATCACGTACCCGAAGCCAAGCTGGTTGAATGCATCGGCCAGGAGCTCATTTTCCTTCTTCCTAACAAGAACTTCAA GCACAGAGCTTATGCCTCTCTGTTCAGGGAACTGGAGGAAACCCTAGCCGACCTGGGTCTGTCTAGCTTCGGCATCTCTG | |
| | ACACACCTCTGGAGGAGATCTTTCTGAAAGTGACTGAGGATTCTGATAGCGGACCACTGTTCGCCGGAGGCGCTCAGCA GAAGAGAGAAAACGTCAATCCTAGACACCCTTGTCTGGGACCGAGAGAGAAGGCCGGCCAGACCCCTCAAGATTCCAA TGTGTGTAGCCCTGGCGCCCCCGCTGCTCACCCAGAGGGCCAGCCTCCTCCTGAACCTGAATGCCCCGGCCCCCAGCTG AACACCGGCACTCAGCTGGTCCTGCAGCACGTGCAGGCCCTGCTGGTGAAGCGCTTCCAGCACACAATCCGCAGTCATA AAGACTTCCTCGCTCAGATCGTGCTCCCGGCTACATTCGTGTTTCTGGCCCTCATGCTGTCTATCGTGATCCCACCTTTCG GCGAATACCCTGCGCTGACCCTGCACCCCTGGATATACGGTCAGCAATACACCTTTTTCAGCATGGACGAGCCCGGAAG CGAGCAGTTCACCGTGCTGGCCGATGTGCTGCTGAACAAGCCTGGCTTCGGCAACCGGTGCCTGAAAGAAGGATGGCTG CCAGAGTACCCCTGCGGCAATAGCACACCTTGGAAGACCCCTAGCGTGTCCCCTAACATCACCCAGCTGTTTCAAAAAC AGAAATGGACCCAAGTGAATCCTTCACCTTCATGCAGATGTTCTACACGTGAAAAGCTGACCATGTTACCCGAGTGTCC TGAAGGAGCTGGCGGCCTTCCTCCTCCCCAGAGGACCCAGAGAAGCACCGAAATCCTGCAGGACCTGACAGACCGGAA CATCTCCGACTTTCTTGTCAAGACCTACCCTGCCCTGATTAGGAGTAGCCTGAAATCTAAGTTTTGGGTTAATGAACAGC GGTACGGCGGCATCAGCATCGGCGGTAAGCTGCCCGTGGTGCCTATTACAGGCGAGGCCCTGGTGGGCTTCCTCAGTGA TTTGGGACGGATAATGAACGTGAGTGGCGGCCCAATCACACGGGAAGCCAGCAAAGAGATCCCTGATTTCCTGAAGCA CCTGGAAACAGAGGATAATATAAAGGTGTGGTTCAACAACAAAGGCTGGCACGCCCTGGTGAGTTTCCTCAACGTAGCC CACAACGCTATCCTGAGAGCCTCTCTACCTAAGGACAGAAGCCCCGAAGAGTACGGCATTACCGTGATCAGCCAGCCCC TAAACCTGACAAAGGAACAGCTGAGCGAGATTACCGTCCTGACAACCTCCGTTGACGCGGTGGTGGCGATCTGCGTGAT CTTTTCTATGAGCTTCGTGCCTGCCAGCTTCGTGCTGTACCTGATCCAGGAGAGAGTGAACAAGAGCAAGCACCTGCAA | |
| | TTTATTTCTGGCGTGAGCCCAACCACCTACTGGGTGACCAACTTCCTGTGGGACATCATGAACTACAGCGTGTCTGCCGG CCTGGTGGTAGGCATCTTCATCGGCTTCCAGAAGAAGGCCTACACCTCTCCTGAGAATCTGCCTGCTCTCGTGGCCCTGC TGCTGCTGTACGGCTGGGCCGTGATCCCCATGATGTACCCCGCTAGCTTCTTATTTGACGTGCCCTCAACAGCATACGTG GCCTTATCCTGCGCCAACCTGTTTATAGGAATCAATTCTAGCGCCATCACGTTCATCCTAGAACTGTTCGAGAACAACCG GACACTGCTGCGATTCAACGCCGTCCTGCGGAAGCTCCTGATTGTGTTCCCTCATTTCTGTCTGGGCAGAGGGCTGATCG ACCTGGCCCTGAGCCAAGCCGTGACCGATGTGTACGCGAGATTCGGCGAGGAGCACTCCGCCAACCCTTTCCACTGGGA CCTGATCGGCAAGAATCTGTTCGCCATGGTTGTGGAGGGTGTGGTGTACTTCCTGCTTACACTGCTGGTGCAGAGACACT TCTTCCTGTCCCAGTGGATTGCCGAACCCACCAAGGAACCCATCGTCGACGAGGATGACGACGTGGCCGAGGAGAGAC AGAGAATCATCACCGGCGGGAACAAAACCGACATCCTGAGACTCCACGAGCTGACAAAGATCTATCCTGGCACAAGCA GCCCCGCCGTGGATCGGCTGTGTGTGGGCGTCAGACCTGGCGAGTGCTTCGGCCTGCTGGGCGTGAACGGCGCCGGCAA GACCACAACCTTCAAGATGCTGACCGGCGACACCACGGTGACCTCCGGCGACGCCACAGTGGCCGGCAAAAGCATCCT CACAAACATCTCCGAAGTGCACCAGAACATGGGCTACTGCCCCCAGTTTGATGCTATAGACGAGCTGTTGACCGGTAGA GAACACCTGTACCTGTACGCCAGACTGAGAGGCGTGCCTGCTGAAGAGATCGAAAAGGTGGCAAACTGGAGCATCAAG TCACTGGGCCTGACAGTCTACGCCGACTGCCTGGCCGGCACGTACTCTGGCGGCAATAAGCGGAAACTGAGCACCGCCA TTGCCTTGATTGGATGTCCGCCTCTGGTGCTGCTGGACGAGCCGACAACAGGCATGGACCCCCAAGCCCGGAGAATGCT GTGGAACGTGATCGTGAGCATAATCAGAGAGGGCAGAGCCGTGGTTTTGACTTCTCACAGCATGGAAGAATGTGAAGC GCTCTGCACCCGGCTTGCCATCATGGTCAAAGGCGCCTTCCGGTGCATGGGCACCATTCAGCACCTGAAGTCCAAGTTC | |
| | GGCGACGGCTACATCGTGACAATGAAGATCAAATCTCCCAAGGACGACCTGCTTCCAGATCTGAACCCTGTGGAACAGT TCTTCCAGGGAAATTTCCCTGGAAGCGTGCAGAGAGAGAGACACTACAATATGCTGCAGTTTCAGGTAAGCAGCAGCA GCCTTGCTCGGATCTTTCAGCTGCTGCTTAGCCACAAGGACTCTCTGCTGATCGAAGAGTACAGCGTGACCCAGACAAC GCTTGATCAGGTGTTCGTGAACTTCGCCAAGCAGCAAACCGAGAGCCATGATCTGCCTCTTCACCCACGAGCTGCCGGC GCCAGCAGACAGGCCCAGGACtga | |
| DnaE protein | CLSYETEILTVEYGLLPIGKIVEKRIECTVYSVDNNGNIYTQPVAQWHDRGEQEVFEYCLEDGSLIRATKDHKFMTVDGQMLP IDEIFERELDLMRVDNLPNIKIATRKYLGKQNVYDIGVERDHNFALKNGFIASNCFN | 4 |
| GP41-1 protein | CLDLKTQVQTPQGMKEISNIQVGDLVLSNTGYNEVLNVFPKSKKKSYKITLEDGKEIICSEEHLFPTQTGEMNISGGLKEGMCL YVKEMMLKKILKIEELDERELIDIEVSGNHLFYANDILTHNS | 5 |
| GP41-8 protein | CLSLDTMVVTNGKAIEIRDVKVGDWLESECGPVQVTEVLPIIKQPVFEIVLKSGKKIRVSANHKFPTKDGLKTINSGLKVGDFL RSRAKMCEIFENEIDWDEIASIEYVGVEETIDINVTNDRLFFANGILTHN | 6 |
| NrdJ-1 protein | CLVGSSEIITRNYGKTTIKEVVEIFDNDKNIQVLAFNTHTDNIEWAPIKAAQLTRPNAELVELEIDTLHGVKTIRCTPDHPVYTK NRGYVRADELTDDDELVVAIMEAKTYIGKLKSRKIVSNEDTYDIQTSTHNFFANDILVHN | 7 |
| IMPDH-1 protein | CFVPGTLVNTENGLKKIEEIKVGDKVFSHTGKLQEVVDTLIFDRDEEIISINGIDCTKNHEFYVIDKENANRVNEDNIHLFARW VHAEELDMKKHLLIELEMKFKLKEITSIETKHYKGKVHDLTVNQDHSYNVRGTVVHN | 8 |
| SspGyrB protein | CFSGDTLVALTDGRSVSFEQLVEEEKQGKQNFCYTIRHDGSIGVEKIINARKTKTNAKVIKVTLDNGESIICTPDHKFMLRDGS YKCAMDLTLDDSLMPLHRKISTTEDSGHAMEAVLNYNHRIVNIEAVSETIDVYDIEVPHTHNFALASGVFVHN | 9 |
| Mja-KlbA protein | ALAYDEPIYLSDGNIINIGEFVDKFFKKYKNSIKKEDNGFGWIDIGNENIYIKSFNKLSLIIEDKRILRVWRKKYSGKLIKITTKN RREITLTHDHPVYISKTGEVLEINAEMVKVGDYIYIPKNNTGSAGSAAGSGINLDEVIKVETVDYNGHIYDLTVEDNHTYIAGK NEGFAVSN | 10 |
| NpuSsp protein | CLSYETEILTVEYGSLPIGKIVEKRIECTVYSVDNNGNIYTQPVAQWHDRGEQEVFEYCLEDGSLIRATKDHKFMTVDGQMLP IDEIFERELDLMRVDNLPNGSAGSAAGSGVKVIGRRSLGVQRIFDIGLRQDHNFLLANGAIAAN | 11 |
| HA tag | YPYDVPDYA | 12 |
| Flag tag | DYKDDDDK | 13 |
| Degradation signal ecDHFR | GRKNIILSSQPSTDDRVTWVKSVDEAIAACGDVPEIMVIGGGRVIEQFLPKAQKLYLTHIDAEVEGDTHFPDYEPDDWESVFSE FHDADAQNSHSYCFEILERR | 14 |
| N-terminal of DnaE | CLSYETEILTVEYGLLPIGKIVEKRIECTVYSVDNNGNIYTQPVAQWHDRGEQEVFEYCLEDGSLIRATKDHKFMTVDGQMLP IDEIFERELDLMRVDNLPN | 15 |
| C-terminal of DnaE | IKIATRKYLGKQNVYDIGVERDHNFALKNGFIASNCFN | 16 |
| N-terminal of gp41-1 | CLDLKTQVQTPQGMKEISNIQVGDLVLSNTGYNEVLNVFPKSKKKSYKITLEDGKEIICSEEHLFPTQTGEMNISGGLKEGMCL YVKE | 17 |
| C-terminal of gp41-1 | MMLKKILKIEELDERELIDIEVSGNHLFYANDILTHNS | 18 |
| N-terminal of gp41-8 | CLSLDTMVVTNGKAIEIRDVKVGDWLESECGPVQVTEVLPIIKQPVFEIVLKSGKKIRVSANHKFPTKDGLKTINSGLKVGDFL RSRAK | 19 |
| C-terminal of gp41-8 | MCEIFENEIDWDEIASIEYVGVEETIDINVTNDRLFFANGILTHN | 20 |
| N-terminal of NrdJ-1 | CLVGSSEIITRNYGKTTIKEVVEIFDNDKNIQVLAFNTHTDNIEWAPIKAAQLTRPNAELVELEIDTLHGVKTIRCTPDHPVYTK NRGYVRADELTDDDELVVAI | 21 |
| C-terminal of NrdJ-1 | MEAKTYIGKLKSRKIVSNEDTYDIQTSTHNFFANDILVHN | 22 |
| N-terminal of IMPDH-1 | CFVPGTLVNTENGLKKIEEIKVGDKVFSHTGKLQEVVDTLIFDRDEEIISINGIDCTKNHEFYVIDKENANRVNEDNIHLFARW VHAEELDMKKHLLIELE | 23 |
| C-terminal of IMPDH-1 | MKFKLKEITSIETKHYKGKVHDLTVNQDHSYNVRGTVVHN | 24 |
| N-terminal of SspGyrB | CFSGDTLVALTDGRSVSFEQLVEEEKQGKQNFCYTIRHDGSIGVEKIINARKTKTNAKVIKVTLDNGESIICTPDHKFMLRDGS YKCAMDLTLDDSLMPLHRKISTTEDSGHA | 25 |
| C-terminal of SspGyrB | MEAVLNYNHRIVNIEAVSETIDVYDIEVPHTHNFALASGVFVHN | 26 |
| N-terminal of Mja-KlbA | ALAYDEPIYLSD | 27 |
| C-terminal of Mja-KlbA | GNIINIGEFVDKFFKKYKNSIKKEDNGFGWIDIGNENIYIKSFNKLSLIIEDKRILRVWRKKYSGKLIKITTKNRREITLTHDHPV YISKTGEVLEINAEMVKVGDYIYIPKNNTGSAGSAAGSGINLDEVIKVETVDYNGHIYDLTVEDNHTYIAGKNEGFAVSN | 28 |
| N-terminal of NpuSsp | CLSYETEILTVEYGSLPIGKIVEKRIECTVYSVDNNGNIYTQPVAQWHDRGEQEVFEYCLEDGSLIRATKDHKFMTVDGQMLP IDEIFERELDLMRVDNLPNGSAGSAAGSGVKVIGRRSLGVQRIFDIGLRQ | 29 |
| C-terminal of NpuSsp | DHNFLLANGAIAAN | 30 |
| Coding nucleotide sequence of N-terminal of DnaE | TGTCTCTCCTACGAGACAGAGATCCTGACAGTGGAATATGGACTGCTGCCCATTGGAAAAATCGTGGAAAAGCGGATCG AGTGCACCGTCTACAGCGTTGATAACAACGGCAACATCTACACCCAACCTGTGGCCCAGTGGCACGATAGAGGCGAAC AGGAGGTGTTCGAGTACTGCCTGGAAGATGGCTCTCTGATCAGAGCCACCAAGGACCACAAGTTCATGACCGTGGACG GCCAGATGCTGCCAATCGACGAGATCTTCGAGAGAGAGCTGGACCTGATGAGAGTGGACAATCTGCCTAAT | 31 |
| Coding nucleotide sequence of C-terminal of DnaE | ATCAAGATCGCTACAAGGAAGTACCTGGGCAAACAGAACGTGTACGACATCGGCGTGGAACGGGACCACAACTTCGCC CTGAAGAACGGCTTCATCGCCAGCAACTGCTTTAAC | 32 |
| Coding nucleotide sequence of N-terminal of gp41-1 | TGTCTGGATCTGAAGACCCAGGTCCAGACCCCTCAGGGCATGAAAGAGATCAGCAACATCCAGGTGGGCGACCTGGTG CTGTCTAATACCGGCTACAACGAGGTGCTGAACGTGTTCCCCAAGAGCAAGAAAAGCTATAAGATCACCCTGGAA GATGGCAAGGAAATCATCTGCAGCGAGGAACACCTGTTTCCTACACAAACAGGCGAGATGAACATCTCTGGCGGACTG AAGGAAGGCATGTGCCTGTACGTGAAGGAG | 33 |
| Coding nucleotide sequence of C-terminal of gp41-1 | ATGATGCTGAAAAAGATCCTGAAGATTGAGGAACTGGACGAGAGAGAGCTGATCGACATCGAGGTGTCCGGAAATCAC CTGTTCTACGCCAACGACATCCTCACCCACAACAGC | 34 |
| Coding nucleotide sequence of N-terminal of gp41-8 | TGTCTGAGCCTGGACACCATGGTGGTGACAAACGGCAAGGCCATCGAGATCAGAGATGTGAAGGTTGGAGATTGGCTG GAATCTGAATGCGGCCCCGTGCAGGTGACCGAGGTGCTGCCTATCATCAAGCAGCCTGTCTTTGAGATTGTGCTGAAAA GCGGAAAAAAGATCCGGGTGTCCGCTAATCACAAGTTCCCAACAAAGGACGGCCTCAAGACCATCAACAGCGGCCTGA AGGTGGGCGACTTCCTGAGAAGCAGAGCCAAG | 35 |
| Coding nucleotide sequence of C-terminal of gp41-8 | ATGTGCGAAATCTTCGAGAACGAGATTGATTGGGACGAAATCGCCAGCATCGAGTACGTGGGCGTGGAAGAGACAATC GACATCAACGTGACCAACGACAGACTGTTTTTCGCCAATGGCATCCTGACCCACAAC | 36 |
| Coding nucleotide sequence of N-terminal of NrdJ-1 | TGTCTGGTTGGATCTAGCGAGATTATCACAAGAAACTACGGCAAGACCACAATCAAGGAGGTGGTGGAAATCTTCGAC AACGACAAGAACATCCAGGTGCTGGCCTTCAACACCCACACCGATAATATCGAGTGGGCTCCTATCAAAGCCGCTCAGC TGACCAGACCAAACGCCGAGCTCGTGGAACTGGAAATCGACACCCTGCACGGCGTGAAGACAATCCGGTGCACCCCTG ACCACCCCGTGTACACCAAGAATAGAGGCTACGTGCGGGCCGACGAGCTGACAGACGATGATGAGCTGGTCGTGGCCA TC | 37 |
| Coding nucleotide sequence of C-terminal of NrdJ-1 | ATGGAAGCCAAGACATACATCGGCAAGCTGAAAAGCAGAAAGATCGTGTCTAACGAGGACACCTACGACATCCAGACC AGCACCCACAACTTCTTCGCCAATGATATCCTGGTGCACAAC | 38 |
| Coding nucleotide sequence of N-terminal of IMPDH-1 | TGTTTTGTGCCTGGAACACTGGTGAATACCGAGAACGGCCTGAAGAAAATCGAGGAAATCAAGGTGGGCGATAAGGTG TTCTCTCATACAGGCAAGCTGCAGGAGGTGGTGGACACCCTGATCTTCGACCGGGACGAGGAAATCATCAGCATCAACG GCATCGACTGCACCAAGAACCACGAGTTCTACGTGATCGATAAGGAAAACGCTAATAGAGTGAACGAGGACAACATCC ACCTCTTCGCCAGATGGGTCCACGCCGAGGAACTGGATATGAAAAAGCACCTGCTGATTGAGCTGGAA | 39 |
| Coding nucleotide sequence of C-terminal of IMPDH-1 | ATGAAATTCAAGCTGAAGGAAATCACCTCTATCGAGACAAAGCACTACAAGGGCAAGGTGCACGATCTGACCGTGAAC CAGGACCACAGCTACAACGTCAGAGGCACCGTGGTGCATAAT | 40 |
| Coding nucleotide sequence of N-terminal of SspGyrB | TGTTTAGCGGCGACACCCTGGTCGCCCTGACAGATGGAAGAAGCGTGTCCTTCGAGCAGCTGGTGGAAGAGGAAAAA CAGGGCAAGCAGAACTTCTGCTACACCATCCGGCACGACGGCTCTATCGGCGTGGAAAAGATCATTAACGCTAGAAAG ACCAAGACAAACGCCAAGGTGATCAAGGTGACCCTGGATAATGGCGAGAGCATCATCTGCACCCCTGATCATAAGTTC ATGCTGCGGGACGGAAGCTACAAGTGCGCCATGGACCTGACACTCGACGACAGCCTGATGCCCCTGCACAGAAAAATC AGCACCACCGAGGACTCTGGCCACGCC | 41 |
| Coding nucleotide sequence of C-terminal of SspGyrB | ATGGAAGCTGTGCTGAACTACAACCACAGAATCGTGAACATCGAAGCCGTCAGCGAGACAATCGATGTGTACGACATC GAGGTGCCTCACACCCACAACTTCGCCCTGGCCTCTGGCGTGTTCGTGCATAAT | 42 |
| Coding nucleotide sequence of N-terminal of Mja-KlbA | GCCCTGGCCTACGACGAGCCTATCTACCTGAGCGAT | 43 |
| Coding nucleotide sequence of C-terminal of Mja-KlbA | GGAAATATCATCAATATTGGCGAGTTCGTGGATAAGTTCTTCAAGAAATACAAGAACAGCATCAAAAAGGAAGATAAT GGCTTCGGCTGGATCGACATCGGCAACGAAAACATCTACATCAAGTCTTTCAACAAGCTGAGCCTGATTATCGAGGACA AAAGAATCCTGAGAGTGTGGCGGAAGAAGTACAGCGGAAAACTCATCAAGATCACAACCAAGAATAGACGGGAAATC ACCCTGACACACGACCATCCTGTGTACATCAGCAAGACCGGCGAAGTGCTGGAAATCAACGCCGAGATGGTGAAGGTG GGAGATTACATCTATATCCCCAAGAACAACACCGGCTCTGCCGGCAGCGCCGCTGGCAGCGGCATCAACCTGGACGAG GTGATCAAGGTCGAGACAGTGGACTACAACGGCCACATCTACGACCTGACCGTTGAGGACAACCACACCTACATCGCC GGCAAGAACGAGGGCTTTGCCGTGTCCAAC | 44 |
| Coding nucleotide sequence of N-terminal of NpuSsp | TGTCTGAGCTACGAGACAGAGATCCTGACCGTGGAATATGGCAGCCTGCCTATTGGCAAAATCGTGGAAAAGCGGATC GAGTGCACCGTCTACAGCGTGGACAACAACGGAAATATCTACACCCAACCTGTGGCCCAGTGGCACGATAGAGGCGAA CAGGAGGTGTTCGAGTACTGCCTGGAAGATGGATCTCTGATCCGGGCCACAAAGGACCACAAGTTCATGACCGTGGAC GGCCAGATGCTGCCAATCGACGAGATCTTTGAGCGGGAACTGGACCTGATGAGAGTGGATAACCTGCCCAACGGCAGC GCCGGCTCTGCCGCTGGCAGCGGCGTTAAGGTGATCGGAAGACGCTCCCTCGGCGTGCAGAGAATCTTCGACATCGGCC TGAGACAG | 45 |
| Coding nucleotide sequence of C-terminal of NpuSsp | GACCACAACTTCCTGCTGGCCAACGGCGCCATCGCCGCTAAT | 46 |
| OPEFS promoter | gggcagagcgcacatcgcccacagtccccgagaagttggggggaggggtcggcaattgaaccggtgcctagagaaggtggcgcggggtaaactgggaaagtgatgtcgtgtactggctccgcc tttttcccgagggtgggggagaaccgtatataagtgcagtagtcgccgtgaacgttctttttcgcaacgggtttgccgccagaacacagGTAAGTATCAAGGTTACAAGACAG GTTTAAGGAGACCAATAGAAACTGGGCTTGTCGAGACAGAGAAGACTCTTGCGTTTCTGATAGGCACCTATTGGTCTTA CTGACATCCACTTTGCCTTTCTCTCCACAG | 47 |
| hGRK1 promoter | GGGCCCAGAAGCCTGGTGGTTGTTTGTCCTTCTCAGGGGAAAAGTGAGGCGGCCCCTTGGAGGAAGGGGCCGGGCAG AATGATCTAATCGGATTCCAAGCAGCTCAGGGGATTGTCTTTTTCTAGCACCTTCTTGCCACTCCTAAGCGTCCTCCGTG ACCCCGGCTGGGATTTAGCCTGGTGCTGTGTCAGCCCCGGGCTCCCAGGGGCTTCCCAGTGGTCCCCAGGAACCCTCGA CAGGGCCAGGGCGTCTCTCTCGTCCAGCAAGGGCAGGGACGGGCCACAGGCCAAGGGCgtaagtttagtctttttgtcttttatttcaggtcccg gatccggtggtggtgcaaatcaaagaactgctcctcagtggatgttgcctttacttctag | 48 |
| smCAG promoter | CCATTGACGTCAATAATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACGTCAATGACGGTAAA TGGCCCGCCTGGCATTATGCCCAGTACATGACCTTATGGGACTTTCCTACTTGGCAGTACATCTACGTATTAGTCATCGC TATTACCATGGTCGAGGTGAGCCCCACGTTCTGCTTCACTCTCCCCATCTCCCCCCCCTCCCCACCCCCAATTTTGTATTT ATTTATTTTTTAATTATTTTGTGCAGCGATGGGGGCGGGGGGGGGGGGGGGGCGCGCGCCAGGCGGGGCGGGGCGGGG CGAGGGGCGGGGCGGGGCGAGGCGGAGAGGTGCGGCGGCAGCCAATCAGAGCGGCGCGCTCCGAAAGTTTCCTTTTAT GGCGAGGCGGCGGCGGCGGCGGCCCTATAAAAAGCGAAGCGCGCGGCGGGCGG | 49 |
| SV40 terminator | aacttgtttattgcagcttataatggttacaaataaagcaatagcatcacaaatttcacaaataaagcatttttttcactgcattctagttgtggtttgtccaaactcatcaatgtatctta | 50 |
| BGH terminator | ctgtgccttctagttgccagccatctgttgtttgcccctcccccgtgccttccttgaccctggaaggtgccactcccactgtcctttcctaataaaatgaggaaattgcatcgcattgtctgagtaggtgtcat tctattctggggggtggggtggggcaggacagcaagggggaggattgggaagacaatagcaggcatgctggggatgcggtgggctctatgg | 51 |
| WPRE-3 terminator | ataatcaacctctggattacaaaatttgtgaaagattgactggtattcttaactatgttgctccttttacgctatgtggatacgctgctttaatgcctttgtatcatgctattgcttcccgtatggctttcattttctcct ccttgtataaatcctggttagttcttgccacggcggaactcatcgccgcctgccttgcccgctgctggacaggggctcggctgttgggcactgacaattccgtgg | 52 |
| CMV enhancer | CCATTGACGTCAATAATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACGTCAATGACGGTAAA TGGCCCGCCTGGCATTATGCCCAGTACATGACCTTATGGGACTTTCCTACTTGGCAGTACATCTACGTATTAGTCATCGC TATTACCATGG | 53 |
| Intron of rhesus beta herpesvirus-3 (MHV-3) | GTAAGTATCAAGGTTACAAGACAGGTTTAAGGAGACCAATAGAAACTGGGCTTGTCGAGACAGAGAAGACTCTTGCGT TTCTGATAGGCACCTATTGGTCTTACTGACATCCACTTTGCCTTTCTCTCCACAG | 54 |
| SV40 intron | gtaagtttagtctttttgtcttttatttcaggtcccggatccggtggtggtgcaaatcaaagaactgctcctcagtggatgttgcctttacttctag | 55 |
| WPRE-SV40 terminator | aatcaacctctggattacaaaatttgtgaaagattgactggtattcttaactatgttgctccttttacgctatgtggatacgctgctttaatgcctttgtatcatgctattgcttcccgtatggctttcattttctcctc cttgtataaatcctggttgctgtctctttatgaggagttgtggcccgttgtcaggcaacgtggcgtggtgtgcactgtgtttgctgacgcaacccccactggttggggcattgccaccacctgtcagctcct ttccgggactttcgctttccccctccctattgccacggcggaactcatcgccgcctgccttgcccgctgctggacaggggctcggctgttgggcactgacaattccgtggtgttgtcggggaaatcatc gtcctttccttggctgctcgcctgtgttgccacctggattctgcgcgggacgtccttctgctacgtcccttcggccctcaatccagcggaccttccttcccgcggcctgctgccggctctgcggcctcttcc gcgtcttcgccttcgccctcagacgagtcggatctccctttgggccgcctccccgc | 56 |
| PURO CDS amino acid sequence | MTEYKPTVRLATRDDVPRAVRTLAAAFADYPATRHTVDPDRHIERVTELQELFLTRVGLDIGKVWVADDGAAVAVWTTPES VEAGAVFAEIGPRMAELSGSRLAAQQQMEGLLAPHRPKEPAWFLATVGVSPDHQGKGLGSAVVLPGVEAAERAGVPAFLET SAPRNLPFYERLGFTVTADVEVPEGPRTWCMTRKPGA | 57 |
| PURO CDS coding nucleotide sequence | atgaccgagtacaagcccacggtgcgcctcgccacccgcgacgacgtccccagggccgtacgcaccctcgccgccgcgttcgccgactaccccgccacgcgccacaccgtcgatccggaccg ccacatcgagcgggtcaccgagctgcaagaactcttcctcacgcgcgtcgggctcgacatcggcaaggtgtgggtcgcggacgacggcgccgcggtggcggtctggaccacgccggagagcg tcgaagcgggggcggtgttcgccgagatcggcccgcgcatggccgagttgagcggttcccggctggccgcgcagcaacagatggaaggcctcctggcgccgcaccggcccaaggagcccgc gtggttcctggccaccgtcggcgtctcgcccgaccaccagggcaagggtctgggcagcgccgtcgtgctccccggagtggaggcggccgagcgcgccggggtgcccgccttcctggagacctc cgcgccccgcaacctccccttctacgagcggctcggcttcaccgtcaccgccgacgtcgaggtgcccgaaggaccgcgcacctggtgcatgacccgcaagcccggtgcctga | 58 |
| EF1A promoter | cgtgaggctccggtgcccgtcagtgggcagagcgcacatcgcccacagtccccgagaagttggggggaggggtcggcaattgaaccggtgcctagagaaggtggcgcggggtaaactgggaa agtgatgtcgtgtactggctccgcctttttcccgagggtgggggagaaccgtatataagtgcagtagtcgccgtgaacgttctttttcgcaacgggtttgccgccagaacacaggtaagtgccgtgtgtg gttcccgcgggcctggcctctttacgggttatggcccttgcgtgccttgaattacttccacctggctccagtacgtgattcttgatcccgagctggagccaggggcgggccttgcgctttaggagcccct tcgcctcgtgcttgagttgaggcctggcctgggcgctggggccgccgcgtgcgaatctggtggcaccttcgcgcctgtctcgctgctttcgataagtctctagccatttaaaatttttgatgacctgctgc gacgctttttttctggcaagatagtcttgtaaatgcgggccaggatctgcacactggtatttcggtttttgggcccgcggccggcgacggggcccgtgcgtcccagcgcacatgttcggcgaggcggg gcctgcgagcgcggccaccgagaatcggacgggggtagtctcaagctggccggcctgctctggtgcctggcctcgcgccgccgtgtatcgccccgccctgggcggcaaggctggcccggtcgg caccagttgcgtgagcggaaagatggccgcttcccggccctgctccagggggctcaaaatggaggacgcggcgctcgggagagcgggcgggtgagtcacccacacaaaggaaaagggccttt ccgtcctcagccgtcgcttcatgtgactccacggagtaccgggcgccgtccaggcacctcgattagttctggagcttttggagtacgtcgtctttaggttggggggaggggttttatgcgatggagtttc cccacactgagtgggtggagactgaagttaggccagcttggcacttgatgtaattctccttggaatttggcctttttgagtttggatcttggttcattctcaagcctcagacagtggttcaaagtttttttcttcc atttcaggtgtcgtgaacacgtg | 59 |
| Rma protein | CLAGDTLITLADGRRVPIRELVSQQNFSVWALNPQTYRLERARVSRAFCTGIKPVYRLTTRLGRSIRATANHRFLTPQGWKRV DELQPGDYLALPRRIPTASMAAACPELRQLAQSDVYWDPIVSIEPDGVEEVFDLTVPGPHNFVANDIIAHN | 60 |
| N-terminal of Rma | CLAGDTLITLADGRRVPIRELVSQQNFSVWALNPQTYRLERARVSRAFCTGIKPVYRLTTRLGRSIRATANHRFLTPQGWKRV DELQPGDYLALPRRIPTAS | 61 |
| C-terminal of Rma | MAAACPELRQLAQSDVYWDPIVSIEPDGVEEVFDLTVPGPHNFVANDIIAHN | 62 |
| Coding nucleotide sequence of N-terminal of Rma | tgtctggctggcgatactctcattaccctggccgatggacgacgagtgcctattagagaactggtgtcacagcagaatttttccgtgtgggctctgaatcctcagacttaccgcctggagagggctaga gtgagtagagctttctgtaccggcatcaaacctgtgtaccgcctcaccactagactggggagatccattagggccactgccaaccaccgatttctcacacctcagggctggaaacgagtcgatgaact ccagcctggagattacctggctctgcctaggagaatccctactgcctcc | 63 |
| Coding nucleotide sequence of C-terminal of Rma | atggcggcggcgtgcccggaactgcgtcagctggcgcagagcgatgtgtattgggatccgattgtgagcattgaaccggatggcgtggaagaagtgtttgatctgaccgtgccgggcccgcataac tttgtggcgaacgatattattgcgcataac | 64 |
| B-globin-polyA terminator | aataaaggaaatttattttcattgcaatagtgtgttiggttttttgtat | 65 |
| sNRP1 terminator | aataaaatacgaaatgtgacaga | 66 |
| enOPEFS promoter | CCATTGACGTCAATAATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACGTCAATGACGGTAAA TGGCCCGCCTGGCATTATGCCCAGTACATGACCTTATGGGACTTTCCTACTTGGCAGTACATCTACGTATTAGTCATCGC TATTACCATGGgggcagagcgcacatcgcccacagtccccgagaagttggggggaggggtcggcaattgaaccggtgcctagagaaggtggcgcggggtaaactgggaaagtgatg tcgtgtactggctccgcctttttcccgagggtgggggagaaccgtatataagtgcagtagtcgccgtgaacgttctttttcgcaacgggtttgccgccagaacacagGTAAGTATCAAGG TTACAAGACAGGTTTAAGGAGACCAATAGAAACTGGGCTTGTCGAGACAGAGAAGACTCTTGCGTTTCTGATAGGCACC TATTGGTCTTACTGACATCCACTTTGCCTTTCTCTCCACAG | 67 |
| enhGRK1 promoter | CCATTGACGTCAATAATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACGTCAATGACGGTAAA TGGCCCGCCTGGCATTATGCCCAGTACATGACCTTATGGGACTTTCCTACTTGGCAGTACATCTACGTATTAGTCATCGC TATTACCATGGGGGCCCCAGAAGCCTGGTGGTTGTTTGTCCTTCTCAGGGGAAAAGTGAGGCGGCCCCTTGGAGGAAGG GGCCGGGCAGAATGATCTAATCGGATTCCAAGCAGCTCAGGGGATTGTCTTTTTCTAGCACCTTCTTGCCACTCCTAAGC GTCCTCCGTGACCCCGGCTGGGATTTAGCCTGGTGCTGTGTCAGCCCCGGGCTCCCAGGGGCTTCCCAGTGGTCCCCAGG AACCCTCGACAGGGCCAGGGCGTCTCTCTCGTCCAGCAAGGGCAGGGACGGGCCACAGGCCAAGGGCgtaagtttagtctttttgtctt ttatttcaggtcccggatccggtggtggtgcaaatcaaagaactgctcctcagtggatgttgcctttacttctag | 68 |
| hGRK1-b-globin-intron promoter | GGGCCCAGAAGCCTGGTGGTTGTTTGTCCTTCTCAGGGGAAAAGTGAGGCGGCCCCTTGGAGGAAGGGGCCGGGCAG AATGATCTAATCGGATTCCAAGCAGCTCAGGGGATTGTCTTTTTCTAGCACCTTCTTGCCACTCCTAAGCGTCCTCCGTG ACCCCGGCTGGGATTTAGCCTGGTGCTGTGTCAGCCCCGGGCTCCCAGGGGCTTCCCAGTGGTCCCCAGGAACCCTCGA CAGGGCCAGGGCGTCTCTCTCGTCCAGCAAGGGCAGGGACGGGCCACAGGCCAAGGGCGTAAGTATCAAGGTTACAAG ACAGGTTTAAGGAGACCAATAGAAACTGGGCTTGTCGAGACAGAGAAGACTCTTGCGTTTCTGATAGGCACCTATTGGT CTTACTGACATCCACTTTGCCTTTCTCTCCACAG | 69 |
| enhGRK1-b-globin-intron promoter | CCATTGACGTCAATAATGACGTATGTTCCCATAGTAACGCCAATAGGGACTTTCCATTGACGTCAATGGGTGGAGTATTT ACGGTAAACTGCCCACTTGGCAGTACATCAAGTGTATCATATGCCAAGTACGCCCCCTATTGACGTCAATGACGGTAAA TGGCCCGCCTGGCATTATGCCCAGTACATGACCTTATGGGACTTTCCTACTTGGCAGTACATCTACGTATTAGTCATCGC TATTACCATGGGGGCCCCAGAAGCCTGGTGGTTGTTTGTCCTTCTCAGGGGAAAAGTGAGGCGGCCCCTTGGAGGAAGG GGCCGGGCAGAATGATCTAATCGGATTCCAAGCAGCTCAGGGGATTGTCTTTTTCTAGCACCTTCTTGCCACTCCTAAGC GTCCTCCGTGACCCCGGCTGGGATTTAGCCTGGTGCTGTGTCAGCCCCGGGCTCCCAGGGGCTTCCCAGTGGTCCCCAGG AACCCTCGACAGGGCCAGGGCGTCTCTCTCGTCCAGCAAGGGCAGGGACGGGCCACAGGCCAAGGGCGTAAGTATCAA GGTTACAAGACAGGTTTAAGGAGACCAATAGAAACTGGGCTTGTCGAGACAGAGAAGACTCTTGCGTTTCTGATAGGC ACCTATTGGTCTTACTGACATCCACTTTGCCTTTCTCTCCACAG | 70 |

## Claims

1. An expression cassette combination, comprising the first expression cassette and the second expression cassette, wherein the first expression cassette is capable of expressing a fusion protein of an N-terminal truncated form of ABCA4 protein and the N-terminal of an intein, and the second expression cassette is capable of expressing a fusion protein of a C-terminal truncated form of ABCA4 protein and the C-terminal of the intein, wherein when expressed, the N-terminal truncated form of ABCA4 protein expressed by the first expression cassette and the C-terminal truncated form of ABCA4 protein expressed by the second expression cassette can form a complete full-length ABCA4 protein through the cleavage function of the N-terminal and C-terminal of the intein.

2. The expression cassette combination according to claim 1, wherein the intein is selected from the group consisting of: GP41-1 (SEQ ID NO: 5), DnaE (SEQ ID NO: 4), GP41-8 (SEQ ID NO: 6), NrdJ-1 (SEQ ID NO: 7), IMPDH-1 (SEQ ID NO: 8), SspGyrB (SEQ ID NO: 9) and Rma (SEQ ID NO: 60), preferably GP41-1.

3. The expression cassette combination according to any one of claims 1-2, wherein the complete ABCA4 protein is a human ABCA4 protein (SEQ ID NO: 1), and the N-terminal truncated form of the ABCA4 protein expressed by the first expression cassette and the C-terminal truncated form of the ABCA4 protein expressed by the second expression cassette are the corresponding N-terminal truncated form and C-terminal truncated form obtained by truncating the complete ABCA4 protein at the following amino acid (Cys) sites starting from the N-terminus of the ABCA4 protein, respectively: 1224, 1140, 1150 or 1188, preferably 1188 or 1224, i.e., the N-terminal truncated form and the C-terminal truncated form are selected from the following combinations:
the amino acids at positions 1-1223 and 1224-2273 from the N-terminus of human ABCA protein (SEQ ID NO: 1);
the amino acids at positions 1-1139 and 1140-2273 from the N-terminus of human ABCA protein (SEQ ID NO: 1);
the amino acids at positions 1-1149 and 1150-2273 from the N-terminus of human ABCA protein (SEQ ID NO: 1); or
the amino acids at positions 1-1187 and 1188-2273 from the N-terminus of human ABCA protein (SEQ ID NO: 1).

4. The expression cassette combination according to claim 2, wherein the N-terminal of intein and the C-terminal of the intein are selected from the group consisting of:
1) The N-terminal of GP41-1 (SEQ ID NO: 17) and the C-terminal of GP41-1 (SEQ ID NO: 18);
2) The N-terminal of DnaE (SEQ ID NO: 15) and the C-terminal of DnaE (SEQ ID NO: 16);
3) The N-terminal of GP41-8 (SEQ ID NO: 19) and the C-terminal of GP41-8 (SEQ ID NO: 20);
4) The N-terminal of NrdJ-1 (SEQ ID NO: 21) and the C-terminal of NrdJ-1 (SEQ ID NO: 22);
5) The N-terminal of IMPDH-1 (SEQ ID NO: 23) and the C-terminal of IMPDH-1 (SEQ ID NO: 24);
6) The N-terminal of SspGyrB (SEQ ID NO: 25) and the C-terminal of SspGyrB (SEQ ID NO: 26); and
7) The N-terminal of Rma (SEQ ID NO: 61) and the C-terminal of Rma (SEQ ID NO: 62).

5. The expression cassette combination according to any one of claims 1-4, wherein the intein is GP41-1, and the N-terminal truncated form and C-terminal truncated form of the ABCA4 protein are the corresponding N-terminal truncated form and C-terminal truncated form obtained by truncating at the 1224th amino acid (Cys) site starting from the N-terminus of the ABCA4 protein, i.e., the 1st to 1223rd amino acids and the 1224th to 2273rd amino acids of the human ABCA4 protein (SEQ ID NO: 1) starting from the N-terminus.

6. The expression cassette combination according to any one of claims 1-4, wherein the intein is GP41-1, and the N-terminal truncated form and C-terminal truncated form of the ABCA4 protein are the corresponding N-terminal truncated form and C-terminal truncated form obtained by truncating at the 1188th amino acid (Cys) site starting from the N-terminus of the ABCA4 protein, i.e., the 1st to 1187th amino acids and the 1188th to 2273rd amino acids of the human ABCA4 protein (SEQ ID NO: 1) starting from the N-terminus.

7. The expression cassette combination according to any one of claims 1-6, wherein the codons for expressing the N-terminal truncated form of the ABCA4 protein and the C-terminal truncated form of the ABCA4 protein are optimized codons, and their combined sequence is the coding sequence of the full-length ABCA4 protein as shown in SEQ ID NO: 3.

8. The expression cassette combination according to any one of claims 1-7, wherein the C-terminus of the fusion protein expressed by the first expression cassette is linked to the first tag, and/or the C-terminus of the fusion protein expressed by the second expression cassette is linked to the second tag, wherein the first tag and the second tag are different, and the first tag and the second tag are independently selected from the group consisting of: hemagglutinin (HA) tag, Flag tag, V5 tag, Myc tag, His tag, 1D4 tag (Rho1D4) and combinations thereof, preferably the first tag is a hemagglutinin (HA) tag (SEQ ID NO: 12) and/or the second tag is a Flag tag (SEQ ID NO: 13).

9. The expression cassette combination according to any one of claims 1-8, wherein the N-terminus or C-terminus of the intein is directly or indirectly connected to a degradation signal, preferably the degradation signal is selected from polypeptides related to DHFR (dihydrofolate reductase), FKBP (FK506 binding protein), FRB (FKBP-rapamycin binding protein) and PDE5 (phosphodiesterase type 5), and degradation signals of endoplasmic reticulum-associated degradation (ERAD) pathway, more preferably the degradation signal is ecDHFR (SEQ ID NO: 14).

10. The expression cassette combination according to any one of claims 1-9, wherein the first expression cassette and/or the second expression cassette comprise(s) a promoter and a terminator for expressing the fusion protein, and optionally comprise(s) an enhancer and an intron for enhancing the expression of the fusion protein and operably linked to the fusion protein.

11. The expression cassette combination according to claim 10, wherein the promoter is a constitutive promoter or an inducible promoter, preferably selected from the group consisting of: CAG promoter (hybrid CMV early enhancer/chicken β-actin promoter, also known as CAGGS promoter, CB promoter or CBA promoter), human β-actin promoter, small CBA (smCBA) promoter, CBS promoter or CBh promoter, elongation factor 1α short (EFS) promoter, elongation factor 1α (EF-1α) promoter, CMV promoter, PGK promoter, UBC promoter, GUSB promoter, UCOE promoter, VMD2 (also known as BEST1) promoter, ABCA4 self-promoter, RPE65 promoter, OPEFS promoter, hGRK1 promoter or smCAG promoter, more preferably OPEFS promoter (SEQ ID NO: 47), hGRK1 promoter (SEQ ID NO: 48), smCAG promoter (SEQ ID NO: 49), enOPEFS promoter (SEQ ID NO: 67), enhGRK1 (enRK) promoter (SEQ ID NO: 68), hGRK1-b-globin-intron promoter (SEQ ID NO: 69) or enhGRK1-b-globin-intron promoter (SEQ ID NO: 70).

12. The expression cassette combination according to claim 10 or 11, wherein the terminator is selected from the group consisting of: SV40 terminator (SEQ ID NO: 50), BGH terminator (SEQ ID NO: 51), WPRE terminator (such as WPRE-3 terminator (SEQ ID NO: 52)), WPRE-SV40 terminator (SEQ ID NO: 56), WPRE-BGH terminator, B-globin-polyA terminator (SEQ ID NO: 65) or sNRP1 terminator (SEQ ID NO: 66).

13. The expression cassette combination according to any one of claims 10-12, wherein the first expression cassette sequentially comprises, from the 5'-end: an optional enhancer, a promoter, an optional intron, a nucleotide coding sequence of the N-terminal truncated form of the ABCA4 protein, a nucleotide coding sequence of the N-terminal of intein, an optional degradation signal, a nucleotide coding sequence of an optional tag, and a terminator, and/or the second expression cassette sequentially comprises, from the 5'-end: an optional enhancer, a promoter, an optional intron, a nucleotide coding sequence of the C-terminal of the intein, an optional degradation signal, a nucleotide coding sequence of the C-terminal truncated form of the ABCA4 protein, a nucleotide coding sequence of an optional tag, and a terminator.

14. The expression cassette combination according to any one of claims 1-13, wherein the first expression cassette and/or the second expression cassette exist(s) in a form selected from the group consisting of: an isolated nucleic acid molecule, a liposome, and/or an exosome.

15. The expression cassette combination according to any one of claims 1-14, wherein the first expression cassette and/or the second expression cassette exist(s) in the form of a plasmid(s).

16. The expression cassette combination according to any one of claims 1-15, wherein the first expression cassette and/or the second expression cassette is/are a viral vector(s).

17. The expression cassette combination according to claim 16, wherein the viral vector is an adeno-associated virus (AAV) of the same or different serotypes, and the AAV is selected from the group consisting of: AAV1, AAV2, AAV3, AAV4, AAV5, AAV6, AAV7, AAV8, AAV9, AAV10, AAV11, AAV12, AAV2/5, AAV2/8, AAV2/1, AAV2/9, AAV2/6, AAV2/4, AAV2/6, AAV5/2, AAV8/1, AAV8/2, AAV2/7, AAV2/12, AAV2/10, AAV-DJ, AAV-DJ/8, AAV-DJ/9, AAV8-Y447,733F or AAVtYF, preferably AAV5, AAV8, AAV8-Y447,733F, AAVtYF or AAV2.7m8.

18. The expression cassette combination according to any one of claims 1-17, wherein:
1) The promoter is hGRK1, the truncation site of the N-terminal truncated form and the C-terminal truncated form is 1188, the intein is Rma, and the terminator is SV40;
2) The promoter is enRK, the truncation site of the N-terminal truncated form and the C-terminal truncated form is 1188, the intein is GP41-1, and the terminator is SV40;
3) The promoter is enRK, the truncation site of the N-terminal truncated form and the C-terminal truncated form is 1188, the intein is Rma, and the terminator is B-globin-polyA;
4) The promoter is enRK, the truncation site of the N-terminal truncated form and the C-terminal truncated form is 1188, the intein is GP41-1, and the terminator is B-globin-polyA;
5) The promoter is hGRK1, the truncation site of the N-terminal truncated form and the C-terminal truncated form is 1188, the intein is GP41-1, and the terminator is SV40;
6) The promoter is enRK, the truncation site of the N-terminal truncated form and the C-terminal truncated form is 1150, the intein is GP41-1, and the terminator is SV40; or
7) The promoter is enRK-b-globin intron, the truncation site of the N-terminal truncated form and the C-terminal truncated form is 1188, the intein is GP41-1, and the terminator is B-globin-polyA.

19. A kit comprising the expression cassette combination according to any one of claims 1-18.

20. Use of the expression cassette combination according to any one of claims 1-18 or the kit according to claim 19 in the preparation of a medicament for treating a disease, wherein the disease comprises diseases caused by ABCA4 mutations.

21. Use according to claim 20, wherein the disease comprises hereditary retinal diseases.

22. Use according to any one of claims 20-21, wherein the disease comprises hereditary macular degeneration diseases, age-related macular degeneration, retinitis pigmentosa, and/or cone-rod dystrophy.
